Europäisches Patentamt

European Patent Office

Office européen des brevets

(11) Publication number: **0 499 112 A1**

## EUROPEAN PATENT APPLICATION

(21) Application number: **92101732.3**

(22) Date of filing: **03.02.92**

(51) Int. Cl.⁵: **C12P 21/08**, C12N 5/20,
C12N 15/06, G01N 33/577,
G01N 33/68, A61K 39/395

(30) Priority: **11.02.91 US 653936**

(43) Date of publication of application:
**19.08.92 Bulletin 92/34**

(84) Designated Contracting States:
**AT BE CH DE DK ES FR GB GR IT LI LU MC
NL PT SE**

(71) Applicant: **F. HOFFMANN-LA ROCHE AG**
**Postfach 3255**
**CH-4002 Basel(CH)**

(72) Inventor: **Chizzonite, Richard Anthony**
**104 Richards Road**
**South Kent, Connecticut 06785(US)**
Inventor: **Hakimi, John**
**210 Country Ridge Road**
**Scarsdale, N.Y: 10583(US)**
Inventor: **Kochan, Jarema Peter**
**7 Crestmont Road**
**Verona, N.J. 07044(US)**

(74) Representative: **Mezger, Wolfgang, Dr. et al**
**Grenzacherstrasse 124 Postfach 3255**
**CH-4002 Basel(CH)**

(54) **Antibodies against an IgE receptor.**

(57) The present invention is directed to a monoclonal antibody or a fragment thereof capable of binding to at least one epitope of the α-subunit of the human high affinity Fc IgE receptor, hybridoma cells secreting such a monoclonal antibody, a process for the preparation of such a hybridoma cell and for the preparation of such a monoclonal antibody or fragment thereof, pharmaceutical compositions containing such an antibody or fragment thereof and the use of such an antibody or fragment thereof for therapy, diagnosis and purification purposes.

The instant invention is directed to a monoclonal antibody or a fragment thereof capable of specifically binding to at least one epitope of the $\alpha$ subunit of the human high affinity Fc IgE receptor, a process for their preparation and their use.

The high affinity Fc receptor for immunoglobulin E (IgE), known as FcERI, is found on mast cells and basophils. Crosslinking of this receptor by IgE and antigen induces cellular degranulation and release of preformed mediators such as histamine and serotonin (1). It is the release of these mediators which contributes to the allergic state whereby leukotriene and platelet activating factor synthesis are also stimulated.

Therefore FcERI plays a central role in the allergic response (1). In the rat this receptor consists of three different subunits: an IgE binding $\alpha$ chain, one $\beta$ chain and two $\gamma$ chains (1-12). Previous studies have demonstrated that high affinity cell surface IgE binding is dependent on the coexpression of these three subunits (12, 13). In the human system at least the $\alpha$ and $\gamma$ subunits must be present for high affinity cell surface binding (15, 16). The functional contribution of the $\beta$ and/or $\gamma$ subunits at present is unknown.

Complementary DNA clones coding for the rat $\alpha$ (9,10), $\beta$ (11), and $\gamma$ (12) subunits, for the mouse $\alpha$, $\beta$ and $\gamma$ subunits (13) and for the human $\alpha$ (10,14) and $\gamma$ (15) subunits have been isolated. Transfection of the cDNA's coding for either the human $\alpha$ and the rat or human $\gamma$ subunits (14,16) or a chimeric human $\alpha$ subunit alone (17) into heterologous mammalian cells results in cell lines capable of binding human IgE with high affinity.

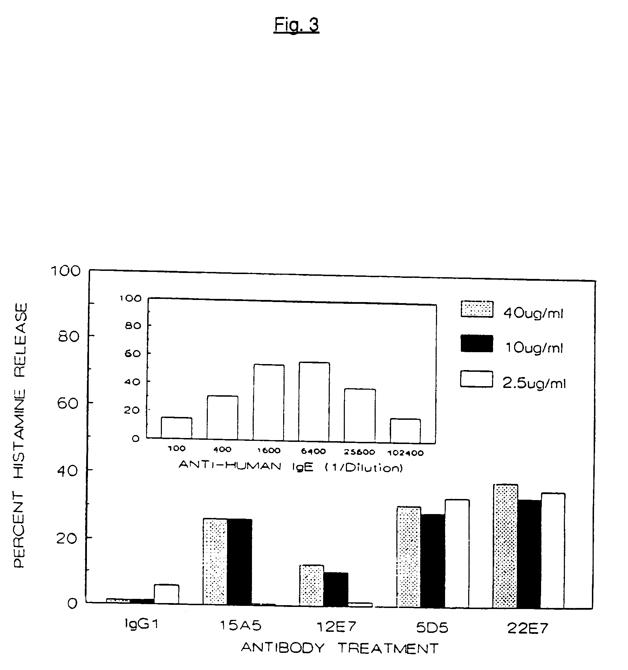

The cDNAs predict proteins with a signal peptide of 23 (rat and mouse), or 25 (human) amino acids, an extracellular domain of 180-181 amino acids, a transmembrane domain of 20-21 amino acids and a cytoplasmic domain of 22 (rat), 25 (mouse) or 33 (human) amino acids. The three $\alpha$ subunits are members of the immunoglobulin superfamily (9,10,13,14) and are most homologous with each other and the $\alpha$ subunit of the mouse Fc$\gamma$RIIa receptor (9,10,13,34). From comparison to other immunoglobulin superfamily members, the sequence for the human $\alpha$ subunit predicts two disulfide bonded loops which encompass amino acid sequences 51-93 and 132-176 of the full length $\alpha$ subunit sequence (Figure 3) (10).

Previous reports have described the development and characterization of monoclonal antibodies specific for high affinity immunoglobulin receptors, for example the rat FcERI receptor (35-37) and the human FcGRI receptor (39). Additionally, one report has described the isolation of a monoclonal antibody that reacts with human basophils (38). Since the binding of this monoclonal antibody to human basophils was blocked by human IgE, it cannot be excluded that this antibody might recognize the high affinity Fc IgE receptor (38). However, reference (38) does not disclose that this monoclonal antibody binds to any subunit of this receptor.

Up until this time it was believed that in order to have surface expression it was necessary to have the $\alpha$ subunit of the human FcERI complexed with additional subunits of FcERI (12). However, Hakimi et al. [J. Biol. Chem. 265, 22079-22081 (1990)] show that modifying the $\alpha$ subunit to construct a hybrid gene which incorporates cytoplasmic or both the cytoplasmic and transmembrane regions of other receptors to allow for cell surface expression of the $\alpha$ subunit [such as the IL-2 receptor, the low affinity IgE receptor (CD23), the murine erythropoietin receptor, the human IgG Fc receptor Fc GRII, etc.] results in surface expression of the $\alpha$ subunit which will also bind IgE with high affinity, and allows for stable expression of this activity in permanently transfected eukaryotic cells.

The monoclonal antibodies of the present invention are capable of specifically binding to at least one epitope of the $\alpha$ subunit of the human high affinity Fc IgE receptor (FcERI). They provide powerful analytical, diagnostic and therapeutic reagents for the immunoaffinity purification of natural and recombinant $\alpha$-subunit of the human FcERI ("$\alpha$-subunit"), the development of immunoassays, the identification of the active site of the $\alpha$-subunit and provide a therapeutic treatment for allergic diseases (by using the Fab fragment or derivatives to block IgE binding to cellular FcERI receptors on human basophils and mast cells). Monoclonal antibodies which recognize different epitopes on the human $\alpha$-subunit are useful as reagents in a sensitive two-site immunoassay to measure levels of $\alpha$-subunit in biological fluids and from cell extracts.

The monoclonal antibodies of this invention, which are capable of specifically binding to at least one epitope of the $\alpha$ subunit of the human high affinity IgE receptor can be characterized e.g. by inducing histamine release from human basophils, immunoprecipitating labelled $\alpha$ subunit and/or blocking IgE binding to recombinant and natural $\alpha$ subunit.

These monoclonal antibodies can be used as affinity reagents for the purification of natural and recombinant human $\alpha$-subunit, as reagents for enzyme-immunoassays and radioimmunoassays to measure natural and recombinant $\alpha$-subunit in biological fluids, on plasma membranes of human cells, as reagents for a drug screening assays, as reagents to construct sensitive two-site immunoassays to measure $\alpha$-subunit in biological fluids and human cell extracts, as reagents in conjunction with recombinant $\alpha$-subunit in

sensitive capture immunoassays to measure IgE levels in biological fluids and cell extracts, as reagents to identify sites of the $\alpha$-subunit which participate in binding IgE, and, in the form of Fab fragments (of the inhibitory monoclonal antibodies), as therapeutic drugs for the treatment of IgE induced allergic diseases.

"Monoclonal antibodies" as used in the present invention include fragments with the same specificity, such as bivalent and monovalent Fab fragments. As is well known in the art, such fragments can be generated by proteolytic cleavage of an immunoglobulin molecule. For example, cleavage with papain produces two monovalent Fab fragments, while cleavage with pepsin produces one bivalent Fab fragment. These fragments may be screened for receptor binding activity by conventional methods such as the methods described herein for screening whole monoclonal antibodies. Examples are immunoprecipitation, Western blotting, binding inhibition, and other assays described, e.g. in Example 3.

The monoclonal antibodies of the present invention can be of human, or nonhuman mammalian, origin e.g. rat, mice, and rabbit. These antibodies can be of the IgA, IgM, and IgG subtypes, in particular of the $IgG_1$ subtype. Methods for producing such antibodies are well known in the art, and given in the Examples.

The present invention makes use of a chimeric $\alpha$-subunit which is expressed on a host cell surface independent of the other subunits of FcERI. Therefore, the present invention comprises monoclonal antibodies which are capable of binding to one or more epitopes of the chimeric $\alpha$-subunit expressed on the surface of a host cell, such as e.g. a COS cell or a CHO cell, and also to the isolated chimeric $\alpha$ subunit. The chimeric $\alpha$ subunit is a hybrid polypeptide comprised of a functional $\alpha$ subunit amino acid sequence and the transmembrance and cytoplasmic amino acid sequences of a different receptor, such as the IL-2 receptor (described elsewhere herein).

The present invention is also directed to monoclonal antibodies which are capable of binding to the $\alpha$ subunit expressed on the surface of mast cells or basophils, in particular, human mast cells or basophils. IgE, a bivalent immunoglobulin, binds to its cell surface receptor (FcERI) by its Fc portion, or stem. Each of its Fab "arms" is bound to an antigen, and two IgE molecules can crosslink via an antigen, each IgE being bound by one "arm" to the same antigen. This causes the receptors bound to the IgE molecules to aggregate on the cell surface. Aggregation of the receptors stimulates the cell to degranulate and release histamine. It is this process that contributes to the allergic response.

These antibodies may bind to an epitope at or near the site of IgE binding and so block IgE binding. An example of such an antibody is the antibody 15A5. The antibodies may themselves cause receptor aggregation by binding one receptor with each Fab "arm", and stimulate histamine release. This will be the case if whole immunoglobulins or bivalent Fab fragments are used. These antibodies are particularly useful for assays and purifications. A monovalent antibody, i.e. a monovalent Fab fragment, which blocks IgE binding and does not cause aggregation because it has only one binding site and therefore cannot bind to more than one receptor, is useful therapeutically because it can prevent IgE from inducing histamine release without itself inducing histamine release. Also described herein are monoclonal antibodies which bind to an $\alpha$ subunit epitope that is not at or near the IgE binding site, for example the monoclonal antibody 11B4. Such antibodies do not block IgE binding, and do induce histamine release. To determine whether a monoclonal antibody blocks IgE binding, competition assays using labelled IgE can be performed by methods well known in the art to see if the antibody reduces IgE binding to the $\alpha$ subunit. Chimeric $\alpha$ subunit, or host cells expressing chimeric $\alpha$ subunit (both described herein) are particularly useful to provide binding sites for the assay.

Monoclonal antibodies of the present invention can be characterized e.g. by falling into two main categories: 1) antibodies which block radiolabelled IgE binding to FcERI bearing cells (inhibitory class) and 2) those which do not block this ligand-receptor interaction (non-inhibitory class).

The extracellular domain of the human $\alpha$ subunit contains 7 potentially N-linked glycosylation sites (10,14). The native $\alpha$ subunit and the recombinant chimeric $\alpha$ subunit expressed in CHO cells have apparent molecular weights of approximately 50 kD (2,3) and approximately 45 kD (17), respectively, while the predicted molecular weights from their cDNAs are 26 kD (10,14) and 24 kD (17), respectively. Thus most of the seven potential N-linked glycosylation sites appear to be utilized in the mature proteins. Removal of N-linked carbohydrates from the chimeric $\alpha$ subunit by N-glycanase digestion results in a shift in molecular weight from approximately 45 kD to 25 kD. The 25 kD protein is recognized by the monoclonal antibodies of the present invention recognizing the polypeptide core of the $\alpha$ subunit.

Both classes of antibodies bind to the chimeric $\alpha$ subunit with affinities which are nearly equal to or greater than the affinity of IgE for the $\alpha$ subunit. These data indicate that the difference between the two classes of antibodies is not based on affinity but on the epitope bound by each antibody. Five distinct epitopes on the extracellular region of the $\alpha$ subunit of the human FcERI receptor have been identified with the monoclonal antibodies of the present invention: two inhibitory and three non-inhibitory epitopes. To identify the epitopes recognized by the antibodies of the present invention, each antibody was tested in an EIA with synthetic peptides corresponding to the sequence of the $\alpha$ subunit. One inhibitory epitope (sequence 125-140) was identified by antibody 15A5 and one non-inhibitory epitope (sequence 43-58) was identified by antibodies 11B4 and 22E7. The epitopes of other inhibitory and non-inhibitory antibodies were then characterized in a radioreceptor competition assay using radiolabelled 15A5 and 22E7.

All the inhibitory antibodies bind at or at least sterically near the 125-140 sequence since they all compete with $^{125}$I-15A5 for binding to the chimeric $\alpha$ subunit. IgE also competes with $^{125}$I-15A5 for binding to the chimeric $\alpha$ subunit confirming that IgE and 15A5 bind to overlapping domains in the receptor but probably not identical domains. Only one inhibitory antibody, 4B4, blocks $^{125}$I-IgE binding to both the human and rat $\alpha$ subunits and blocks $^{125}$I-15A5 binding to the human $\alpha$ subunit. These data indicate that the 4B4 epitope overlaps but is not identical to the 125-140 epitope of antibody 15A5.

Twenty-two non-inhibitory antibodies completely inhibit $^{125}$I-labelled 22E7 from binding to the chimeric $\alpha$ subunit indicating that most bind at or sterically near the 43-58 sequence of the chimeric $\alpha$ subunit. Three non-inhibitory antibodies do not inhibit radiolabelled 22E7 binding to the chimeric $\alpha$ subunit indicating that these antibodies bind to a different epitope on the protein. One non-inhibitory antibody, 29C6, blocks both $^{125}$I-22E7 and $^{125}$I-15A5 binding to the chimeric $\alpha$ subunit. The $IC_{50}$ for 29C6 inhibition of $^{125}$I-22E7 and $^{125}$I-15A5 binding to the $\alpha$ subunit are 0.18 $\mu$g/ml and >120 $\mu$g/ml, respectively.

Hybridomas which produce the monoclonal antibodies of the present invention are disclosed in this invention, in particular hybridomas which are described in the Examples. Generation of hybridomas is a method well known in the art, and also described in the Examples.

Briefly, in the standard method, an animal such as a mouse, rat, or rabbit is immunized with an antigen, namely a polypeptide comprising at least one epitope of the $\alpha$ subunit, e.g. the whole $\alpha$ subunit itself or in form of a chimeric $\alpha$ subunit as described herein. B-cells are then isolated from the animal, assayed for specificity, and fused to immortal myeloma cells to create hybridomas. The hybridoma cells are diluted and individual cells are aliquoted into receptacles (such as wells in a microtiter plate). The monoclonal antibodies produced by the clones of the individual hybridomas are also screened for specificity. Hybridomas producing antibodies of a desired specificity can also be produced by recombinant DNA methods. Monoclonal antibodies of human origin can be produced by the recombinant method, or can be produced by exposing human lymph cells in culture to $\alpha$ subunit to induce antibody production. After 3-5 days, the antibody-producing cells are fused with myeloma cells, and selection of anti-$\alpha$ subunit antibodies is performed as described supra.

Any conventional screening methods may be used to screen monoclonal antibodies or fragments thereof of the present invention, e.g. those exemplified by Example 3, parts c, e, f, j, k, l, and m. One such method uses the cell described herein which expresses a chimeric $\alpha$ subunit on its surface. For example, monoclonal antibodies can be fluorescently labelled and then added to a slide containing these cells. When the slide is viewed under a fluorescence microscope, antibodies having the described specificity will be bound to the cell and visually detectable. Another method is immunoprecipitation. Isolated $\alpha$ subunit as described herein is detectably labeled by methods well known in the art. Antibodies with the desired specificity will bind to the labelled $\alpha$ subunit to form a complex. The complex can be isolated by adding antibodies coupled to a solid support which bind to the antibodies bound to the $\alpha$ subunit.

Once expressed, the whole antibodies, their dimers, individual light and heavy chains, or other immunoglobulin forms of the present invention can be purified according to standard procedures of the art, including ammonium sulfate precipitation, affinity columns, column chromatography, gel electrophoresis and the like [see e.g. Scopes, R., Protein Purification, Springer-Verlag, N.Y. (1982)]. Substantially pure immunoglobulins of at least about 90 to 95% homogeneity are preferred, and 98-99% or more homogeneity most preferred, for pharmaceutical uses. Once purified, partially or to homogeneity as desired, the polypeptides may then be used therapeutically (including extracorporeally) or in developing and performing assay procedures, immunofluorescent stainings, and the like. [See e.g. Immunological Methods, Vols. I and II, Lefkovits and Pernis, eds., Academic Press, New York, NY (1979 and 1981)].

A method for producing monoclonal antibodies capable of binding to one or more epitopes of the $\alpha$ subunit of the human high affinity IgE receptor, or the chimeric $\alpha$ subunit, entails isolating such antibodies from the medium of the hybridomas described above. The antibodies can also be isolated directly from an immunized animal using the screening method described above.

This invention describes methods for detecting in, or purifying the $\alpha$ subunit from, a sample such as a sample of a biological fluid containing free $\alpha$ subunit, or a sample containing cells and/or membrane-bound $\alpha$ subunit. Monoclonal antibody capable of specifically binding to an epitope or epitope of the $\alpha$ subunit of the human high-affinity IgE receptor, in particular to an epitope or epitopes of the chimeric $\alpha$ subunit, is contacted with the sample under conditions well known in the art such that the monoclonal antibody binds $\alpha$ subunit in the sample. The monoclonal antibody may be in solution or bound to a solid support such as a column or plate or membrane (examples of which are provided herein). When bound to a solid support, $\alpha$ subunit can be eluted and purified by methods known in the art. In solution, the labelled monoclonal antibody can be detected by the means suitable to the label used, to indicate the presence of $\alpha$ subunit in the sample. Another detection method employs unlabelled monoclonal antibody to bind to the $\alpha$ subunit in the sample, which is then contacted with a labelled antibody which binds to the unlabelled antibody. In either method, the amount of labelled antibody, if known, can be used to quantify the amount of $\alpha$ subunit in the sample. Labels are well known in the art. Suitable labels may be radioactive, such as $^{125}$I, fluorescent, biotin or enzymatic, such as peroxidase. Examples of labeling are known in the art and provided e.g. in the Examples.

The present invention includes a method to detect and quantify IgE present in a sample which is capable of binding to the $\alpha$ subunit. Monoclonal antibody capable of specifically binding to the $\alpha$ subunit is coupled to a solid support (such as beads for use in an affinity column, or the surface of a microtiter plate, or other supports well known in the art), and contacted with a known amount of $\alpha$ subunit (the obtaining and quantifying of $\alpha$ subunit are described elsewhere herein). The monoclonal antibody binds to the $\alpha$ subunit to form a complex which is, in its turn, bound to the solid support. This complex is contacted with the sample. In the sample, active IgE, but not denatured or inactive IgE will bind $\alpha$ subunit. The amount of IgE present in the sample can then be quantified by contacting the complex with detectably labelled antibody capable of binding to IgE, and measuring the amount of label. Techniques for producing monoclonal antibodies with selected specificities, such as anti-IgE antibodies, are well known in the art. Also, anti-IgE antibodies are easily obtained from biological suppliers. Although IgE could be detected in an assay using only anti-IgE antibody, such an assay would not be able to discriminate between active and inactive IgE, i.e., IgE capable or incapable of binding $\alpha$ subunit. An assay using $\alpha$ subunit can be used for this purpose, and is also contemplated in this invention.

The present invention includes a pharmaceutical composition comprising a monovalent Fab fragment which is capable of binding specifically to an epitope or epitopes of the $\alpha$-subunit of the human high affinity IgE receptor such that IgE binding is inhibited. The antibody of this invention can be linked to toxins such as diptheria or pseudomonas toxin and used to selectively eliminate receptor bearing cells.

The antibodies and pharmaceutical compositions of the present invention are particularly useful for parenteral administration, i.e., subcutaneously, intramuscularly or intravenously. The compositions for parenteral administration will commonly comprise a solution of the antibody or a cocktail thereof dissolved in an acceptable carrier, preferably an aqueous carrier. A variety of aqueous carriers can be used, e.g., water, buffered water, 0.4% saline, 0.3% glycine and the like. These solutions are sterile and generally free of particulate matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example sodium acetate, sodium chloride, potassium chloride, calcium chloride, sodium lactate, etc. The concentration of antibody in these formulations can vary widely, i.e., from less than about 0.5%, usually at or at least about 1% to as much as 15 or 20% by weight and will be selected primarily based on fluid volumes, viscosities, etc., in accordance with the particular mode of administration selected.

Thus, a typical pharmaceutical composition for intramuscular injection could be made up to contain 1 ml sterile buffered water, and 50 mg of antibody. A typical composition for intravenous infusion could be made up to contain 250 ml of sterile Ringer's solution, and 150 mg of antibody. Actual methods for preparing parenterally administrable compositions will be known or apparent to those skilled in the art and are described in more detail in, for example Remington's Pharmaceutical Science, 15th ed., Mack Publishing Company, Easton, Pennsylvania (1980).

The antibodies of the present invention can be lyophilized for storage and reconstituted in a suitable carrier prior to use. This technique has been shown to be effective with conventional immunoglobulins and lyophilization and reconstitution techniques known in the art can be employed. It will be appreciated by those skilled in the art that lyophilization and reconstitution can lead to varying degrees of antibody activity loss (e.g., with conventional immunoglobulins, IgM antibodies tend to have greater activity loss than IgG antibodies) and that use levels may have to be adjusted to compensate.

The compositions containing the Fab fragments of the present monoclonal antibodies or a cocktail thereof can be administered for prophylactic and/or therapeutic treatments. In therapeutic application, compositions are administered to a patient already suffering from an allergic responses in an amount sufficient to cure or at least partially arrest the disease and its complications. An amount adequate to accomplish this is defined as a "therapeutically effective dose." Amounts effective for this use will depend upon the severity of the allergic response, but generally range from about 1 to about 200 mg of antibody per dose.

In prophylactic applications, compositions containing the present antibodies or a cocktail thereof are administered to a patient not already in a disease state in an amount sufficient to block IgE from the patient's own basophils. Such an amount is defined to be a "prophylactically effective dose." In this use, the precise amounts again depend upon the patient's state of health and general level of circulation antigen specific IgE, from 0.1 to 25 mg. per dose.

The production of one of the possible antigens which can be used in the practice of the present invention, namely the chimeric α subunit entails a number of steps which include the preparation of the genes encoding the hybrid gene sequences, insertion of this gene into a appropriate cloning vehicle to produce a recombinant vector containing the hybrid gene sequence, transfer of the recombinant cloning vehicle into a compatible host organism, selection and growth of such modified hosts that can express the inserted gene sequences, and identification and purification of the gene product.

Preparation of the gene encoding the chimeric α subunit is accomplished by first isolating the appropriate portions of the FcERI α subunit and the cytoplasmic or cytoplasmic and transmembrane regions of the IL-2 receptor.

The nucleotide sequence of the human α subunit is published in Nucleic Acid Research, Vol. 16 (8): 3584 (1988). The nucleotide sequence of the human p55 IL-2 receptor is published in Nature, 311: 631 (1984) and the cytoplasmic and transmembrane regions of the IL-2 receptor span approximately nucleotides 890 to 1030. The nucleotide sequence of the α subunit may be obtained from a variety of sources such as human basophils, mast cells or the Ku812 cell line [Kishi, Leukemia Research, 9, 381 (1985)]. The nucleotide sequence of the IL-2 receptor may also be obtained from a variety of sources such as human T cells, T-cell lines, or peripheral blood lymphocytes.

Preferentially for the use of the present invention the hybrid gene contains nucleotide sequence 121-710 of the human α-subunit to which nucleotides 876-1016 of the IL-2 receptor have been fused. This results in a hybrid gene of the following nucleotide sequence:

```
TACTAAGAGTCTCCAGCATCCTCCACCTGTCTACCACCGAGCATGGGCCTATATTTGAAGCCT

TAGATCTCTCCAGCACAGTAAGCACCAGGAGTCCAT GAAGAAG ATG GCT CCT GCC AGG

ATC CTC CCT ACT CTA CTG TGT GTA GCC TTA CTG TTC TTC GCT CCA GAT

GGC GTG TTA GCA GTC CCT CAG AAA CCT AAG GTC TCC TTG AAC CCT CCA

TGG AAT AGA ATA TTT AAA GGA GAG AAT GTG ACT CTT ACA TGT AAT GGG

AAC AAT TTC TTT GAA GTC AGT TCC ACC AAA TGG TTC CAC AAT GGC AGC

CTT TCA GAA GAG ACA AAT TCA AGT TTG AAT ATT GTG AAT GCC AAA TTT

GAA GAC AGT GGA GAA TAC AAA TGT CAG CAC CAA CAA GTT AAT GAG AGT

GAA CCT GTG TAC CTG GAA GTC TTC AGT GAC TGG CTG CTC CTT CAG GCC

TCT GCT GAG GTG GTG ATG GAG GGC CAG CCC CTC TTC CTC AGG TGC CAT

GGT TGG AGG AAC TGG GAT GTG TAC AAG GTG ATC TAT TAT AAG GAT GGT

GAA GCT CTC AAG TAC TGG TAT GAG AAC CAC AAC ATC TCC ATT ACA AAT

GCC ACA GTT GAA GAC AGT GGA ACC TAC TAC TGT ACG GGC AAA GTG TGG

CAG CTG GAC TAT GAG TCT GAG CCC CTC AAC ATT ACT GTA ATA AAA GCT

CCG CGT GAG AAG T ACC AGG TAG CAG TGG CCG GCT GUG TTT TCC TGC TGA

TCA GCG TCC TCC TCC TGA GTG GGC TCA CCT GGC AGC GGA GAC AGA GTA

AGA GTA GAA CAA TAT TCT AGA AAA CCA AAA GAA CAA GAA TTT CTT GGT

AAG AAG CCG
```

However, in the construction of the hybrid gene it is recognized that various restriction endonucleases will cleave each nucleotide sequence in a different place which may further result in hybrid genes which do not have the exact sequence as described above. So long as the hybrid gene contains at least a nucleotide sequence which codes for a functional polypeptide subsequence of the α subunit (which is any subsequence which will bind IgE) as well as any functional subsequence corresponding to the cytoplasmic or cytoplasmic and transmembrane regions of the IL-2 receptor, it can be used for the purpose of the present invention. Moreover, cytoplasmic or transmembrane regions of any other receptor or transmembrane protein whose sequence can be added to the functional polypeptide subsequence of the α subunit and allow for functional cell surface expression is suitable. Examples of such sequences are numerous as set forth below. In each instance it would be possible to fuse the IgE binding domains of the human α subunit to the transmembrane and cytoplasmic domains of the following proteins (note that in each example the single subunit reaches the cell surface in the absence of any other subunits): the low affinity IgE receptor or CD23 [Kikutani et al., Cell, 47, 657 (1986)]; the murine erythropoietin receptor [D'Andrea et al., Cell, 57, 277 (1989)]; the human IgG Fc Receptor, Fc GRII [Stuart et al., J. Expt. Med., 1668 (1987)]; human poliovirus receptor [Mendelsohn et al., Cell, 56, 855 (1989)]; human CD4 receptor, [Madden et al., Cell, 42, 93 (1985)]; human PDGF receptor [Yarden et al., Nature, 323, 226 (1986)]; human high affinity IgG Fc Receptor [Science, 243, 378)]; human interferon receptor [Aguet et al., Cell, 55, 273)]; human interleukin-1 receptor [Sims et al., Science, 241, 585)]; or CD19 differentiation antigen [Stamenkovic and Seed, J. Expt. Med., 168, 1205)].

The smaller regions of the α subunit which bind IgE and still comprise an epitope to be recognized by the antibodies of the present invention may be systematically determined by sequentially deleting 3 nucleotides at a time from either the 5' or 3' end. The shortest nucleotide sequence which is capable of coding for an IgE binding subunit polypeptide, would define the minimum unit for a functional protein. In the hybrid gene the portion of the natural nucleotide sequence of the α subunit can be substituted to enable the restriction endonuclease chosen to "clip" the nucleotide sequence at the desired position. A BamHI site can be created at positions 119-126 by substituting the natural human α subunit as follows:

```
                 107 110 113 113 119 122 125 128 131 134
natural          ATG GCT CCT GCC ATG GAA TCC CCT ACT CTA


modified                         G  ATC CT


                                 Bam HI site created
```

The human α subunit can be further modified to create a EcoRV restriction endonuclease site at positions 878-890 as follows:

```
                 866 869 872 875 878 881 884 887 890 893 896
natural          CCC AAA AAC AAC TGA TAT AAT TAC TCA AGA AAT


modified                             CGG AT C


                                 EcoRV site created
```

Any minor substitutions of the natural α subunit or the IL-2 receptor nucleotide sequence to facilitate the desired restriction endonuclease cleavage can be prepared by methods known in the art. After isolation of the appropriate nucleotide sequences of both subunits they can be joined by the appropriate ligase. Although recombinant DNA technology is used to synthesize the hybrid gene it is also possible to synthesize it through chemical synthesis methods which are known in the art.

Once the hybrid gene has been constructed it may be introduced into any convenient expression vector, phage, or plasmid in any manner known in the art [see for example, in the Laboratory Manual, Sambrook et al. "Molecular Cloning", Cold Spring Harbor Laboratory, (1989)].

General methods for the preparation of such recombinant DNA molecules are also known in the art [see e.g. U.S. Patent No. 4,237,224 and U.S. Patent No. 4,304,863].

Methods for expressing DNA sequences which code for hybrid proteins as used for the purpose of the present invention include transforming an appropriate host with a recombinant vector having the said DNA sequence operatively linked to the expression control sequence of the vector, culturing the host under appropriate conditions of growth and extracting and isolating the desired hybrid protein from the culture. The skilled artisan may select from these known methods those which are most effective for a particular gene expression and the selection of a particular host for use in this invention is dependent upon a number of factors recognized by the art. These include, for example, compatibility with the chosen expression vector, toxicity of the proteins encoded for the hybrid plasmid, case of recovery of the desired protein, expression characteristics, biosafety and costs.

The COS cells are the preferred hosts for the purpose of the present invention. COS cells are an African green monkey kidney cell line transformed by an origin minus SV40 viral genome and is available from the ATCC under Accession No. CRL 1651. This cell line has been described by Gluzman et al., [Cell 23, 175 (1981)]. CHO cells are also suitable and widely available. Alternative methods for gene amplification applicable to any eukaryotic cell line is the use of the adenosine-deaminase amplification selection system, or the dihydrofolate reductase negative (dhfr) gene amplification system. A variety of prokaryotic and eukaryotic systems are suitable hosts for expressing the processed extracytoplasmic region of the α subunit (residues 26-201).

The hybrid polypeptide used in the practice of the present invention can have the amino acid sequence:

```
            MET Ala Pro Ala Met Glu Ser Pro Thr Leu Leu Cys Val
      Ala Leu Leu Phe Phe Ala Pro Asp Gly Val Leu Ala Val Pro Gln
      Lys Pro Lys Val Ser Leu Asn Pro Pro Trp Asn Arg Ile Phe Lys
      Gly Glu Asn Val Thr Leu Thr Cys Asn Gly Asn Asn Phe Phe Glu
      Val Ser Ser Thr Lys Trp Phe His Asn Gly Ser Leu Ser Glu Glu
      Thr Asn Ser Ser Leu Asn Ile Val Asn Ala Lys Phe Glu Asp Ser
      Gly Glu Tyr Lys Cys Gln His Gln Gln Val Asn Glu Ser Glu Pro
      Val Tyr Leu Glu Val Phe Ser Asp Trp Leu Leu Leu Gln Ala Ser
      Ala Glu Val Val Met Glu Gly Gln Pro Leu Phe Leu Arg Cys His
      Gly Trp Arg Asn Trp Asp Val Tyr Lys Val Ile Tyr Tyr Lys Asp
      Gly Glu Ala Leu Lys Tyr Trp Tyr Glu Asn His Asn Ile Ser Ile
      Thr Asn Ala Thr Val Glu Asp Ser Gly Thr Tyr Tyr Cys Thr Gly
      Lys Val Trp Gln Leu Asp Tyr Glu Ser Glu Pro Leu Asn Ile Thr
      Val Ile Lys Ala Pro Arg Glu Lys Tyr Gln Val Ala Val Ala Gly
      Lys Val Phe Leu Leu Ile Ser Val Leu Leu Leu Ser Gly Leu Thr
      Trp Gln Arg Arg Gln Arg Lys Ser Arg Arg Thr Ile
```

The hybrid protein for the purpose of the present invention may be synthesized by utilizing the general solid phase methods of Merrifield, Journal of American Chemical Society, 85, 2149 (1963).

The hybrid polypeptides may be produced by recombinant methods by inserting the hybrid gene sequence into the appropriate recombinant vector, and transforming a host as set forth in Culler, Methods in Enzymology 152, 684-704 (1987). The transformant is then grown under appropriate culture conditions to yield the corresponding hybrid polypeptides.

In the following the preparation of a chimeric α subunit which can be used e.g. as an antigen for the preparation of the antibodies of the present invention is described in more detail.

The nucleotide sequence of α subunit of FcERI can be modified at positions 119-126 and 884-890 as follows using site directed mutagenesis [Morinaga et al., Bio Tech 2, 636(1984)]. pLJ663 containing the human subunit α cDNA [1197bp subunit α cDNA ligated into the EcoR̄1 site of pGem-4 (Promega): Kochan et al.] 10μg can be digested at 37°C with the restriction enzymes BglII (30 units) and Sty I (30 units) in a final volume of 100 microliters in a buffer containing 150 mM Nacl, 6 mM Tris-HCl pH 8.0., 6 mM MgCl₂ and 6mM 2-mercapto-ethanol. This digestion fragments pLJ663 into 2 fragments corresponding in lengths of approximately 437bp and 3760bp. The 3760bp fragment is isolated by agarose gel electorphoresis and is referred to as the gapped molecule.

pLJ663, 10 µg is digested separately with the restriction enzyme PvuI (30 units) at 37°C in a final volume of 180 microliters in a buffer containing 150mMNaCl, 6mM Tris-HCl pH 8.0, 6mM MgCl₂ and 6 mM 2-mercaptoethanol. This digestion restricts pLJ663 once and generates a linear fragment of approximately 4197 bp. This linear fragment is isolated by agarose gel electrophoresis and is referred to as the linear molecule.

The oligonucleotide mentioned above (50 pmoles) is phosphorylated at its 5' end using polynucleotide kinase (5 units) in a first volume of 20 microliters in a mixture containing 1mM ATP, 50mM Tris-HCl p76, 10mM MgCl₂, 5mM DTT, 0.1mM spermidine, 0.1 mM EDTA for a reaction time of 30 minutes at 37°C, and a further 5 minutes at 70°C and then put on ice.

Site specific mutants can be generated in the following manner: A mixture was formed containing 100 mg of each of the gapped and linear molecules and 12.5 pmoles of the 5' phosphorylated oligonucleotide, in a final volume of 12.5 microliters in a buffer containing 0.16M KCl, 10.4mM Tris-HCl pH 7.4, 7.2mM MgCl₂. This mixture (in a test tube) is incubated in a boiling water bath (volume of 500 ml) for 3 minutes then the container with the boiling water is removed from the hot plate and allowed to cool to room temperature (containing the mixture). Once this mixture is cooled, the final volume is adjusted to 20 microliters with the addition of the following reagents: 4 microliters of 2.5mM dGATC (dGTP,dATP,dTTP, dCTP), 1 microliter of 10 x ligase buffer (45mMNaCl, 45 mMTris-HCl pH 8.0, 45mM MgCl₂, 40mM DTT, 12 mM ATP, 0.4mg/ml Bovine Serum Albumin (Fraction V)), 1 microliter of 10 x NT buffer (.5M Tris-HCl pH 7.2, 0.1M MgSO₄, 1 mM DTT, 500 µg/ml Bovine Serum Albumin (Fraction V), 1 microliter of T4 DNA ligase (400 units) and 0.5 microliters of E.coli DNA polymerase, Large Fragment (Klenow fragment) (2.5 units). This mixture is incubated at 15°C for at least 2 hours, and occasionally for 18 hours.

1 to 10 microliters of this reaction mixture are then used to transform E.coli MC1061, which are identified by their ability to grow on LB plates containing 50 µg/ml ampicillin. Mutated plasmids are identified by their ability to hybridize to a ³²P-labelled oligonucleotide, using standard conditions. Positively hybridizing colonies are confirmed by the presence of a new BamHI restriction site in plasmid DNA isolated from these colonies. This construction is referred to as pLJ663B.

This results in modifications to the natural sequence as follows:

| natural | ATG GCT CCT GCC ATG GAA TCC CCT ACT CTA |
|---------|------------------------------------------|
| modified |                          G  ATC CT      |

This modification creates a nucleotide sequence cleavable by a BamHI restriction endonuclease. A further modification at positions 884-890 creates a nucleotide sequence cleavable by an EcoRV restriction endonuclease. The introduction of the EcoRV is accomplished in a manner similar to that outlined above. The gapped molecule in this example is formed by digesting pLJ663 (10 µg) with the restriction enzyme Pvu II (30 units) at 37°C in a final volume of 100 microliters in a buffer containing 150 mMNacl, 6mM Tris-HCl pH 8.0, 6mM MgCl₂ and 6mM 2-mercapto-ethanol. This digestion fragment pLJ663 into two fragments corresponding in lengths of approximately 600bp and 3600 bp. The 3600 bp fragment is isolated by agarose gel electrophoresis and is referred to as the gapped molecule. The linear molecule is the same as outlined above. The oligonucleotide used in this example is as written below and the procedure for isolating this mutant is as above and positively hybridizing colonies are confirmed by the presence of a new EcoRV restriction site in plasmid DNA isolated from these colonies. This construction is referred to as pLJ663E:

|          | 866 | 869 | 872 | 875 | 878 | 881 | 884 | 887 | 890 | 893 | 896 |
|----------|-----|-----|-----|-----|-----|-----|-----|-----|-----|-----|-----|
| natural  | CCC | AAA | AAC | AAC | TGA | TAT | AAT | TAC | TCA | AGA | AAT |
| modified |     |     |     |     |     |     | CGG | AT  | C   |     |     |

These modifications allow a BamHI-EcoRV fragment to be isolated and inserted into a cloning vector pBC12BI (See Cullen, Methods in Enzymology, 152:684 (1987)) wherein a BamHI-SmaI fragment is deleted.

The BamHI-EcoRV modified fragment of the human α cDNA fragment is inserted into the BamHI-SmaI sites of pBC12BI in the following manner: A BamHI-BsmI fragment, 264bp, from pLJ663B (10 μg of pLJ663B is digested at 37°C with the restriction enzymes BamHI (30 units) and BsmI (30 units) in a final volume of 100 microliters in a buffer containing 150 mM NaCl, 6mM Tris-HCl pH 8.0, 6mM $MgCl_2$ and 6mM 2-mercaptoethanol. The 264 bp fragment is isolated by agarose gel electrophoresis. A 496bp BsmI-EcoRV fragment from pLJ663E (10 μg of pLJ663E) is digested at 37°C with the restriction enzymes BSmI (30 units) and EcoRV (30 units) in a final volume of 200 microliters in a buffer containing 150 mM NaCl, 6mM Tris-HCl pH 8.0, 6mM $MgCl_2$ and 6mM 2-Mercaptoethanol. The 496bp fragment is isolated by agarose gel electrophoresis. The plasmid pBC12BI (10 μλ) is digested with the restriction enzyme Sma I (30 units) at 37°C in a final volume of 100 microliters in a buffer containing 10mM KCl, 10 mM Tris-HCl pH 8.0, 10mM $MgCL_2$ and 6mM2-mercaptoethanol. It is subsequently digested with BamHI (30 units) at 37°C in a final volume of 100 microliters in a buffer containing 150 mM NaCl, 6mM Tris-HCl pH 8.0, 6mM$MgCl_2$ and 6 mM 2-mercaptoethanol. These digestions result in the production of 2 fragments, and the larger approximate 3,500 bp fragment is isolated by agarose gel electrophoresis. These three fragments are added in equimolar ratios in a mixture of final volume 10 microliters in a buffer containing 4.5mM NaCl, 4.5mM Tris-HCl pH 8.0, 4.5mM $MgCl_2$, 4mM DTT, 1.2mM ATP, 40 μg/ml Bovine Serum Albumin and T4 DNA ligase (400 units). The fragments are allowed to ligate overnight at 4°C. E. coli. MC1061 is transformed with the ligation mixture and positive colonies are identified by hybridizing to a nick-translated human α subunit cDNA probe. DNA is isolated from the positive colonies, and the presence of a 760bp BamHI-EcoRV fragment confirmed. Several positives are isolated and one of these is referred to as pLJ1101. This vector, pLJ1101 expresses very high levels of the α subunit but very few of the molecules reach the cell surface or would bind IgE.

A BamHI-Sca I fragment comprising the following nucleotide sequence 121-710 is isolated from pLJ1101:

The plasmid pLJ1101 (10 μg) is digested with the restriction enzyme BamHI (30 units) in a final volume of 100 microliters in a buffer containing 100 mM NaCl, 6mM Tris-HCl pH 8.0, 6mM $MgCl_2$ and 6mM 2-mercaptoethanol to completion. Subsequently the restriction enzyme ScaI (3 units) is added so that a partial digest occurred. A 589bp BamHI-ScaI fragment (nucleotides 121-710 of the human α subunit) is then isolated by agarose gel electrophoresis.

```
                            .....  GAA TCC CCT ACT CTA CTG TGT GTA GCC
      TTA CTG TTC TTC GCT CCA GAT GGC GTG TTA GCA GTC CCT CAG AAA CCT
      AAG GTC TCC TTG AAC CCT CCA TGG AAT AGA ATA TTT AAA GGA GAG AAT
      GTG ACT CTT ACA TGT AAT GGG AAC AAT TTC TTT GAA GTC AGT TCC ACC
      AAA TGG TTC CAC AAT GGC AGC CTT TCA GAA GAG ACA AAT TCA AGT TTG
      AAT ATT GTG AAT GCC AAA TTT GAA GAC AGT GGA GAA TAC AAA TGT CAG
      CAC CAA CAA GTT AAT GAG AGT GAA CCT GTG TAC CTG GAA GTC TTC AGT
      GAC TGG CTG CTC CTT CAG GCC TCT GCT GAG GTG GTG ATG GAG GGC CAG
      CCC CTC TTC CTC AGG TGC CAT GGT TGG AGG AAC TGG GAT GTG TAC AAG
      GTG ATC TAT TAT AAG GAT GGT GAA GCT CTC AAG TAC TGG TAT GAG AAC
      CAC AAC ATC TCC ATT ACA AAT GCC ACA GTT GAA GAC AGT GGA ACC TAC
      TAC TGT ACG GGC AAA GTG TGG CAG CTG GAC TAT GAG TCT GAG CCC CTC
```

AAC ATT ACT GTA ATA AAA GCT CCG CGT GAG AAG .....

This sequence is ligated to a RsaI-BamHI fragment from the IL-2 receptor which comprises nucleotides 876-1016. The plasmid pIL-2R (20 μg) [Hakimi et al., Journal of Biol. Chem., 262 17336-17341 (1987)] is digested with the restriction enzymes BamHI (30 units) and RsaI (30 units) in a final volume of 200 microliters in a buffer containing 100mM NaCl, 6mM Tris-HCl pH 8.0, 6mM $MgCl_2$ and 6mM 2-mercaptoethanol to completion. A 141bp RsaI-BamHI fragment (nucleotides 876-1016 of the p55 II-2 receptor cDNA) is isolated by agarose gel electrophoresis:

```
                                                              A  CCAGGTAGCA
GTGGCCGGCT  GTGTTTTCCT  GCTGATCAGC  GTCCTCCTCC  TGAGTGGGCT  CACCTGGCAG
CGGAGACAGA  GGAAGAGTAG  AAGAACAATC  TAGAAAACCA  AAAGAACAAG  AATTTCTTGG
TAAGAAGCCG
```

The BamHI-ScaI $\alpha$ subunit fragment (589bp), the RsaI-BamHI p55 fragment (141bp) and pBC12BI digested with BamHI, are ligated together in a reaction containing equimolar ratios of all 3 DNA fragments. Correctly ligated plasmid vectors are verified by restriction enzymes analysis. One of these plasmids, pLJ1275, expresses high levels of the $\alpha$ subunit which reach the cell surface and bind IgE, when transfected into COS cells as described by Cullen [Methods in Enzymology 152, 684-704 (1987)].

The human $\alpha$ subunit cDNA (14) can be modified by site directed mutagenesis (46) such that a BamHI site is introduced at nucleotides 120-125, and an EcoRV site is introduced at nucleotides 879-884. The resulting BamHI-EcoRV fragment of the modified cDNA is isolated and cloned into the BamHI-EcoRV fragment of the modified cDNA is isolated and cloned into the BamHI-SmaI sites of pBC12BI (47). This results in an in-frame fusion where the initiation codons and the first five amino acids of the preproinsulin gene are fused to amino acids 6-257 of the human $\alpha$ subunit. This particular construction pLJ1101 results in high level expression when transfected into COS cells as judged by immunofluorescent staining of permeabilized cells using antipeptide antisera 81-103 and 160-197.

The vector containing the chimeric form of the $\alpha$ subunit is constructed by ligating the BamHI-ScaI fragment of the modified $\alpha$subunit (nucleotides 121-710) to an RsaI-BamHI fragment of the IL-2 receptor (nucleotides 876-1016) (48). This ligated fragment is then inserted into BamHI digested pBC12BI. The resulting vector, pLJ1275, directs the synthesis of chimeric $\alpha$ subunit encoding amino acids 6-201 of the human $\alpha$ subunit (corresponding to the signal peptide and extracellular domain) and amino acids 240-271 (corresponding to the transmembrane and cytoplasmic domains) of the p55 IL-2 receptor.

Amplification of the chimeric $\alpha$ subunit expression is achieved by stepwise increasing concentrations of 0.1 and 1.0 micromolar amethropterin. Enrichment of cells expressing high receptor numbers is achieved by two rounds of fluorescent activated cell sorting using IgE-biotin and streptavidin-FITC. Stable clones expressing high levels of cell surface IgE binding subunit are then obtained by limiting dilution cloning. Five independent clonal isolates are initially characterized which differed with respect to receptor expression but not with regard to binding affinity. The 3D10 clone is adapted to spinner cultures in Iscove's modified Dulbecco's medium supplemented with 10% fetal calf serum and 80 $\mu$g/$\mu$l gentamycin sulfate.

For immunofluorescent analysis cells can be incubated with biotinylated-IgE (1-3 mg/ml) for 30 minutes in Iscove's modified Dulbecco's medium supplemented with 10% fetal calf serum (complete medium) at 37°C. The cells are washed 4x with complete medium and incubated with streptavidin-FITC or donkey anti-rabbit IgE-FITC (Jackson Immunoresearch) for 30 minutes at 37°C in complete medium. The cells are then washed and either sorted on an Ortho Cytofluorograph 50H or overlaid with 50% glycerol containing 0.25M N-propylgallate and coverslip mounted. The cells are observed on a Leitz Diaplan fluorescent microscope (25 x dry objective) and photographed with Kodak Tri-X film.

For a binding assays IgE can be radiolabelled with $^{125}$I to a specific activity of 1-2 X $10^6$ cpm/$\mu$g protein using chloramine-T (19). The bindability of $^{125}$I-IgE is assessed as previously described by (49) and ranged between 65-75%. Binding assays are performed as described previously (22), and for competition assays, samples at various dilutions are preincubated with the cell suspensions (4 X $10^6$ cells/ml) for 30 min prior to the addition of 250 ng/ml $^{125}$I-IgE. Nonspecific binding is determined by adding a 50-100 fold excess of unlabeled IgE to the assay tubes, and these values are subtracted from experimental values to give specific binding. Kinetics of association and dissociation between IgE and the recombinant receptor are determined by the methods described previously (50, 51, 16). The forward rate constant ($k_1$) is calculated from the equation $k_1 = V_o/I_gE_oR_o$, where $V_o$ represents the initial rate of binding and $IgE_o$ and $R_o$ represent the initial molar concentrations of IgE and receptor, respectively. The dissociation rate constant ($k_{-1}$) is calculated by assuming that the dissociation of a ligand from receptors is a simple first-order decay process. The equilibrium constant ($K_A$) is calculated with the equation:

$$K_A = k_1/k_{-1}.$$

For binding analysis permanent cell lines expressing the chimeric receptor can be established in CHO cells using gene-linked co- amplification technology. To clearly demonstrate that the extracellular domain of the $\alpha$ subunit is sufficient for high affinity IgE binding, the equilibrium association constant is established by independent determinations of the association and dissociation rate constants as previously described for

EP 0 499 112 A1

the human and rodent receptors. The receptor number on the CHO cells is determined by incubating the cells with 10 $\mu$g/ml of $^{125}$I-IgE for 2 h. Receptor density ranged between 4-10 x $10^5$ molecules/cell.

After the invention has been described in general hereinbefore, the following Figures and Examples are intended to illustrate details of the invention, without thereby limiting it in any manner.

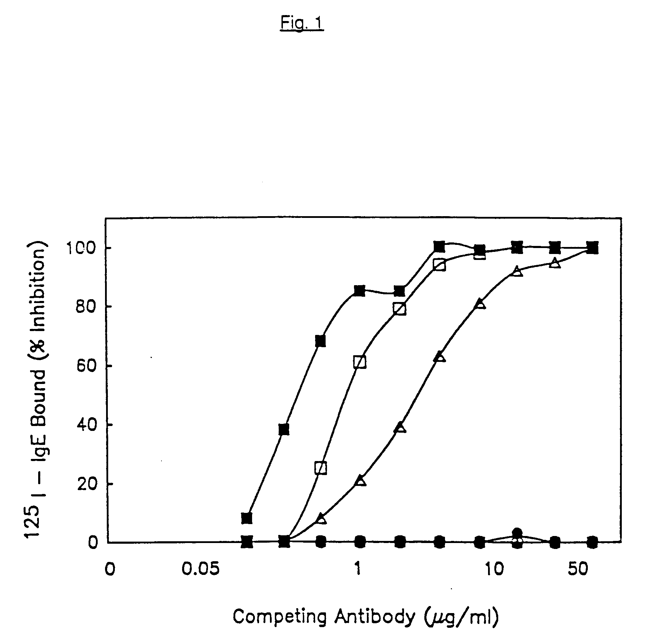

Figure 1. Inhibition of $^{125}$I-IgE (prepared by the chloramine T-method known in the art) binding to CHO-FcERI(chimeric $\alpha$ subunit) cells by monoclonal antibodies. The data are expressed as "%inhibition" of $^{125}$I-IgE binding in the presence of the indicated concentrations of antibody when compared to the total specific binding in the absence of antibody. The %inhibition was determined as described in the Examples for unlabelled IgE ($\square$) and for antibodies 15A5 ($\blacksquare$), 12E7 ($\triangle$), 22E7 ($\bullet$), 5D5 (O), 29C6 (X) and 8C8 ($\blacktriangle$).

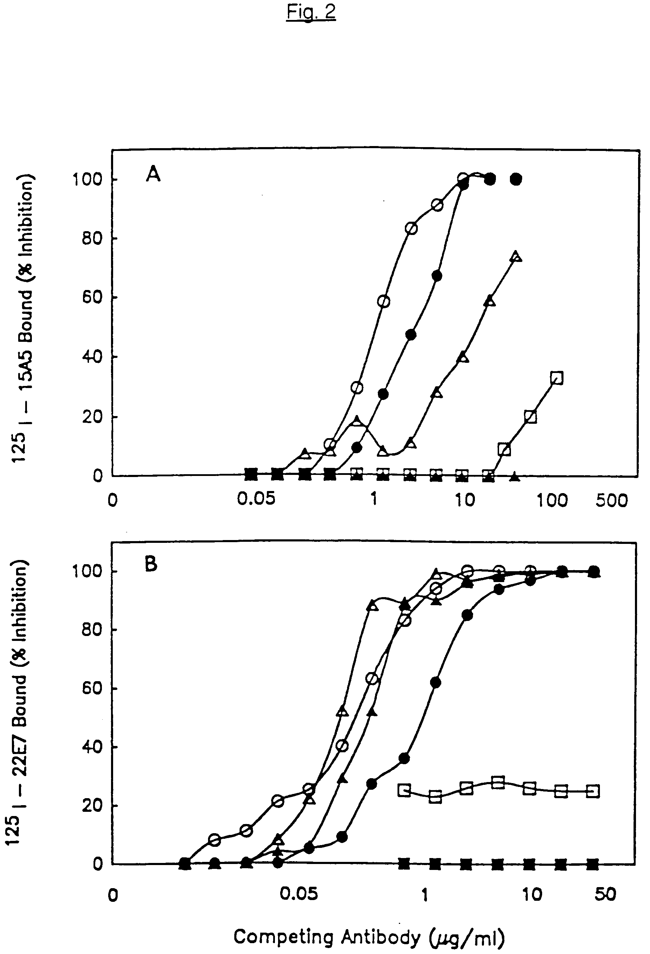

Figure 2. Inhibition of $^{125}$I-15A5 (A) and $^{125}$-22E7 (B) binding to CHO-FcERI(chimeric $\alpha$ subunit) cells by IgE and monoclonal antibodies. The data are expressed as %inhibition of $^{125}$I-15A5 or $^{125}$I-22E7 binding in the presence of the indicated concentrations of antibody when compared to the total specific binding in the absence of antibody. The %inhibition was determined in panel A for unlabelled IgE ($\bullet$) and for antibodies 15A5 ($\square$), 12E7 ($\blacksquare$), 22E7 (O), 5D5 (X), 29C6 ($\square$) and 8C8 ($\blacksquare$) and panel B for antibodies 15A5 ($\square$), 12E7 ($\blacksquare$), 22E7 (O), 5D5 ($\bullet$), 29C6 ($\triangle$) and 8C8 ($\blacktriangle$). Control antibody specific for a non-related protein did not reduce specific binding in either assay.

Figure 3. Stimulation of histamine release from human basophils by treatment with anti-IgE antibodies or antibodies of the present invention. Human peripheral blood basophils were prepared and treated with the indicated concentrations of anti-IgE antibody or antibodies of the present invention as described in the Examples. Histamine release was measured as described (33).

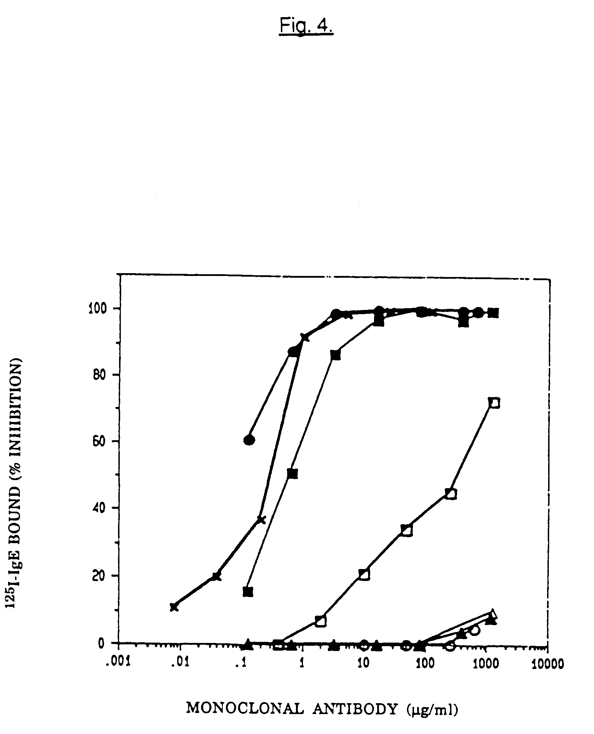

Figure 4. Comparison of the inhibition of $^{125}$I-IgE binding to rat basophilic leukemia (RBL) cells and CHO-FcERI(chimeric $\alpha$ subunit) cells by unlabelled IgE and monoclonal antibodies of the present invention. The data are expressed as %inhibition of $^{125}$I-rat IgE binding to RBL cells, e.g. RBL-1 [ATCC CRL 1378] and $^{125}$I-human IgE binding to CHO-FcERI(chimeric $\alpha$ subunit) cells by unlabelled rat IgE and anti-human $\alpha$ subunit antibodies. The %inhibition was determined as described in the Examples for the indicated concentrations of each antibody. Unlabelled rat IgE (X) and monoclonal antibodies 4B4 ($\square$), 6F7 (O) and control antibody ($\triangle$) on RBL cells. Unlabelled antibodies 4B4 ($\blacksquare$), 6F7 ($\bullet$) and control antibody ($\blacktriangle$) on CHO-FcERI(chimeric $\alpha$ subunit) cells.

## Examples

### Example 1

#### Monoclonal Antibodies to the $\alpha$ subunit

##### a. Purification and Labelling of Human and Rat IgE with $^{125}$I and Biotin

Human myeloma IgE(PS) was purified as described (17,18) and rat monoclonal IgE was purified from the ascites fluid of nu/nu mice bearing Immunocytoma IR 162 as described (19). IgE was biotinylated with biotinyl-epsilon-aminocaproic acid N-hydroxysuccinimide ester (B-X-NHS, CalBiochem) as described (20). Human and rat IgE was radiolabeled with $^{125}$I to a specific activity of 1-2 x $10^6$ cpm/mg protein using chloramine-T (21).

##### b. Purification of Soluble Chimeric $\alpha$ Subunit

CHO cells expressing 4-10 X $10^5$ chimeric $\alpha$ subunit/cell were maintained in Iscove's Modified Dulbecco's Medium (IMDM) containing 10% fetal calf serum (FBS), 1 mM glutamine, 50 $\mu$g gentamycin and 3 x $10^{-6}$ M methotrexate as a selective agent. Cells were harvested, washed with cold phosphate buffered saline (PBS, pH 7.4) and solubilized ($10^8$ cells/ml) in cold PBS containing 10 mM CHAPS, 1 mM EDTA, 40 $\mu$g PMSF and 0.02% NaN$_3$ (10 mM CHAPS/PBS Buffer). The cell extract was centrifuged at 100,000 x g for 70 min to remove cell debris and the solubilized chimeric $\alpha$ subunit was chromatographed on an IgE affinity column.

Human or rat IgE (5 mg) was coupled to 1 ml of Affigel-10 according to the manufacturer's protocol (BioRad Labs, Richmond CA). The IgE affinity resin was washed with five column volumes of 0.2 M HAc, 0.2M NaCl, 2 mM CHAPS, pH 2.8 (acetic acid-CHAPS buffer) followed by reequilibration with 10 mM CHAPS/PBS buffer. Solubilized preparations of chimeric $\alpha$ subunit (1 to 6 x $10^9$ cell equivalents) were applied to the column, washed with 10 mM CHAPS/PBS buffer and 2 mM CHAPS/PBS buffer (2mM CHAPS, 1 mM EDTA, 40 $\mu$g PMSF, 0.02% NaN$_3$ in PBS pH 7.4) and eluted with acetic acid-CHAPS buffer.

12

Each fraction was immediately neutralized with 3 M Tris, pH 9. Fractions containing protein were pooled, dialyzed against 10 mM CHAPS/PBS buffer and assayed in a slot blot assay for active chimeric α subunit.

### c. Slot Blot Assay to Detect Active Chimeric α Subunit

Soluble extracts of CHO-FcERI(chimeric α subunit) cells and affinity purified chimeric α subunit were diluted with an equal volume of 10 mM Tris-glycine buffer, 20% methanol, pH 8.3 and vaccuum filtered onto nitrocellulose strips (0.45 μm) using a commercial slot blotting unit (Schleicher and Schuell, Keene, NH). The nitrocellulose strips were blocked for 1 hr at room temperature in phosphate buffered saline (PBS) containing 2% bovine serum albumine (BSA), incubated with IgE-biotin (0.5 μg) in CHAPS/FBS buffer (10 mM CHAPS, 5% fetal bovine serum, PBS pH 7.4) for 1 hr, washed with PBS containing 0.05% Tween-20 and reacted with streptavidin-peroxidase in CHAPS/FBS buffer (Amersham Corp.) for 30 min. The strips were washed and the chimeric α subunit/biotinylated-IgE complexes were visualized with 4 chloro-1-napthol (0.5 mg/ml in 0.15% $H_2O_2$, 0.5 M NaCl, 50 mM Tris-HCl, pH 7.5) for 30 min.

### d. Labeling of Affinity Purified Chimeric α subunit Protein with $^{125}$I and N-glycanase Treatment of $^{125}$I-chimeric α subunit

Affinity purified chimeric α subunit was labeled for 30 min with 0.2 mCi of Na[125]I using Enzymobead reagent (BioRad Laboratories) according to the manufacturer's protocol. Labeled protein was separated from free iodine on a BioGel P6DG column (BioRad Laboratories), equilibrated with 2 mM CHAPS/PBS, diluted with an equal volume of 2 mM CHAPS/PBS with 1 mg/ml BSA and stored at 4°C.

Radiolabeled chimeric α subunit was dried under vaccuum and resuspended in 0.2M sodium phosphate, 10 mM EDTA, pH 7.0. Digestion with N-glycanase (3 units) (Genzyme, Boston, MA, USA) proceeded for 2 days at 37°C on a shaker and the reaction was stopped with 0.1 M citrate buffer, pH 4.5.

### e. Receptor Binding Assays and Affinity Crosslinking

Binding assays were performed essentially as described (17,22). Briefly, CHO-FcERI(chimeric α subunit) or RBL cells were resuspended to 4 X $10^6$ cells/ml in RPMI 1640, 5 % fetal bovine serum, 5 mM HEPES pH 7.4 (Binding Buffer) and incubated with $^{125}$I-labelled IgE (10 ng/ml to 1 mg/ml). Non-specific binding was determined by adding a 50-100 fold excess of unlabelled IgE to the non-specific assay tubes. Cell bound radioactivity was separated from unbound label by centrifugation through silicone oil (23). For competition assays, antibody samples at various concentrations were preincubated with the cell suspensions for 1 hr at room temperature prior to the addition of $^{125}$I-labelled human IgE (19 ng) or rat IgE (250 ng). Samples were incubated for an additional 1 hr at room temperature and processed as described above. The inhibition of $^{125}$I-IgE to the CHO-FcERI(chimeric α subunit) or RBL cells was determined by comparing the total specific binding in the presence of antibody with the total specific binding in the absence of antibody.

For preparation of soluble complexes of $^{125}$I-IgE crosslinked to chimeric α subunit, cells were incubated with $^{125}$I-IgE, washed with PBS and resuspended in cold PBS, 1 mM MgCl₂, pH 8.3 at a concentration of $10^7$ cells/ml. Disuccinimidyl suberate (DSS) (Pierce Chemical Co.) in dimethyl sulfoxide (MeSO₄) was added to a final concentration of 0.4 mM and the reaction proceeded for 30 min at 4°C with constant agitation. The reaction was quenched and the cells washed with 25 mM Tris-HCl pH 7.5, 0.15 M NaCl, 5 mM EDTA. The cells were solubilized in ice cold 10 mM CHAPS/PBS buffer ($10^7$ cells/ml) as described above. The cell extracts were directly analyzed by SDS/PAGE (24) or were immunoprecipitated with antibodies before analysis by SDS/PAGE.

### f. Preparation, Characterization and Purification of Hybridoma Antibodies

BALB/c mice (Charles River Laboratories, Wilmington, MA) were immunized by the intraperitoneal route (i.p.) with partially purified chimeric α subunit (from 7 X $10^8$ cell equivalents) mixed with an equal volume of Freund's complete adjuvant. Two weeks later, the mice were injected i.p. with a similar amount of purified chimeric α subunit mixed with Freund's incomplete adjuvant. Seven weeks later, one mouse was injected i.p. and i.v. with additional chimeric α subunit (from 5.5 X $10^9$ cell equivalents) on two successive days starting 4 days prior to the cell fusion. Spleen cells were isolated from this mouse and fused with NSO cells (25) at a ratio of 1:1 (spleen cells:NSO cells) with 35% polyethylene glycol (PEG 4000, E. Merck) (26). The fused cells were plated at a density of 5 x $10^4$ cells/well/ml in 48 well plates in IMDM supplemented with 15% FBS, glutamine (2 mM), l-mercaptoethanol (0.1 mM), gentamycin (50 $\mu$g), HEPES (10 mM) and 15% P388D1 supernatant (27). Hybrid supernatants were screened for specific $\alpha$ subunit antibodies in 3 assays: 1) immunoprecipitation of $^{125}$I-labelled chimeric $\alpha$ subunit, 2) immunoprecipitation of the soluble complex of $^{125}$I-IgE crosslinked to chimeric $\alpha$ subunit and 3) inhibition of $^{125}$I-IgE binding to CHO-FcERI(chimeric $\alpha$ subunit) cells. Hybridoma cell lines were cloned by limiting dilution.

For the immunoprecipitation assays (IP assay), hybridoma supernatants or purified antibody samples (20 $\mu$g), IP buffer (4 mM CHAPS/PBS pH 7.4, 1 mg/ml BSA) and either $^{125}$I-labelled chimeric $\alpha$ subunit or soluble complex of $^{125}$I-IgE crosslinked to chimeric $\alpha$ subunit were mixed together and allowed to stand for 30 min. Goat anti-mouse antibodies coupled to agarose beads (Sigma Chemical Co., St. Louis, MO) were added to the tubes and rotated for 2 hr at room temperature. The beads were washed three times with IP buffer and counted. Bound ligand was released by addition of SDS/PAGE sample buffer and heating at 95°C for 3 min. The released proteins were analyzed by SDS/PAGE and autoradiography.

Antibodies were also tested by western blot analysis for binding to denatured solubilized chimeric $\alpha$ subunit (28). Briefly, solubilized proteins were separated by SDS/PAGE under either non-reducing or reducing conditions and electrophoretically transferred to nitrocellulose membranes. The membranes were treated with 1% BSA, PBS pH 7.4 for 1 hr. at 37°C, and subsequently probed with antibody samples diluted in antibody buffer (1% BSA, 50 mM sodium phosphate pH 6.5, 0.5 M NaCL, 0.05% Tween-20) for 2 hr at room temperature. The membranes were washed, reacted with goat anti-mouse IgG coupled to peroxidase and bound antibody visualized with 4-chloro-1-napthol for 30 min at room temperature.

Antibodies were purified from large scale hybridoma cultures by affinity chromatography on Protein G bound to cross-linked agarose according to the manufacturer's protocol (Genex, Gaithersburg, MD, USA).

### g. Binding of Antibodies to Synthetic Peptides

Sixteen overlapping peptides spanning the entire extracellular sequence of the $\alpha$ subunit were prepared by standard solid phase synthesis (29). The purified peptides were dissolved (1 mg/ml) in distilled water or $MeSO_4$. EIA plates were coated with 200 ng of each peptide in 50 mM bicarbonate-carbonate buffer, pH 9.6 and then dried overnight at 37°C. The plates were washed with water, blocked with 1% BSA, PBS pH 7.4 and incubated with antibody for 2 hr at room temperature. The plates were washed with 0.05% Tween-20, PBS pH 7.4, and goat anti-mouse IgG coupled to peroxidase (Boehringer Mannheim Immunochemicals) was added for 2 hr at room temperature. The antibody-peptide complexes were visualized with o-phenylene-diamine (0.4 mg/ml) dissolved in 0.1 M citrate buffer, pH 4.5, containing 0.012% $H_2O_2$ at 492 nm.

### h. Binding Assays with $^{125}$I-labelled Antibodies

Antibodies 15A5, 12E7 and 22E7 were radiolabeled with $^{125}$I by a modification of the Iodogen method (Pierce Chemical Co.). Briefly, 1 mCi of $^{125}$I-Na (Amersham Co.) and 50 $\mu$l of 0.5 M sodium phosphate buffer, pH 7.5 were added to a borosilicate glass tube coated with 50 $\mu$g of Iodogen. After 4 min, antibody (50 $\mu$g) in PBS, pH 7.4 was added and the reaction continued for 8 min at room temperature. Labelled antibody was separated from free $^{125}$I by chromatography on a BioGel P6DG column (BioRad Laboratories). The binding of labelled antibodies to CHO-FcERI(chimeric $\alpha$ subunit) cells was determined as described for $^{125}$I-IgE. Non-specific binding was determined in the presence of 100 fold molar excess of unlabelled antibody or IgE. The equilibrium binding data were analyzed according to Scatchard (30) to estimate the dissociation constant ($K_D$) for the labelled antibody and the number of binding sites per cell. Competition assays with unlabelled purified antibodies and hybridoma supernatants were performed as described for $^{125}$I-IgE, except that $^{125}$I-labelled antibody is added at the end of the 1 hr pre-incubation step. The concentration of antibody which inhibited 50% of the binding of labelled ligand was designated as the $IC_{50}$.

### i. Binding of Antibodies to Native FcERI on Human Basophils

Human basophils were enriched from heparinized peripheral blood by sedimentation in Ficoll-Paque (Pharmacia) as previously described (31). Washed basophils ($1.5 \times 10^5$ cells) were incubated with 2.5-40 mg/ml purified antibodies in PBS containing 1%FBS and 0.02% sodium azide for 20 min at room temperature. The cells were washed and then incubated with goat anti-human IgE labelled with fluorescein and (Fab)$_2$-goat anti-mouse IgG labelled with rhodamine (Tago Labs, Burlingame, CA.). Cells were washed and dispersed on a slide using a Cytospin apparatus (400 rpm for 5 min). The slides were overlaid with 90% glycerol containing 0.22M 1,4-diazo bicyclo (2,2,2) octane (Sigma Chemical Co.) as described previously (32). The cells were observed on a Leitz Diaplan fluorescent microscope and photographed with Kodak Ektachrome 400 film.

Histamine release was performed as described previously (33).

j. Characterization of Monoclonal Antibodies to the α Subunit.

Crude extracts of CHO-FcERI(chimeric α subunit) cells and purified soluble chimeric α subunit were evaluated for specific IgE binding activity by a slot blot assay. Qualitative assessment of the results of the slot blot assay demonstrated that the amount of partially purified chimeric α subunit present in the primary and secondary immunizations was approximately 7 to 28 $\mu$g. For the final immunization, the mouse received approximately 100 $\mu$g of partially purified chimeric α subunit as determined in the slot blot assay. Mice injected with purified chimeric α subunit produced serum antibodies that effectively blocked $^{125}$I-IgE binding to CHO-FcERI(chimeric α subunit) cells and immunoprecipitated both the $^{125}$I-labelled chimeric α subunit and the soluble complex of $^{125}$I-IgE cross-linked to chimeric α subunit. A fusion with spleen cells isolated from one mouse yielded 47 hybridomas secreting monoclonal antibodies (mAb) of the present invention (Table 1). All the antibodies immunoprecipitated a radiolabelled approximately 47 kD protein from the $^{125}$I-labelled chimeric α subunit preparation. The two major radiolabelled proteins of approximately 47 kD and approximately 65 kD present in the $^{125}$I-chimeric α subunit preparation represent labelled chimeric α subunit and bovine serum albumin, respectively. For data see Table 1 wherein numbers 1)-7) have the following meaning:

## Table 1

| mAb Class | IP ASSAY[1] | | | RECEPTOR BINDING ASSAY[2] CHO-FcERI(chimeric α subunit) | | | RBL | PEPTIDE EIA[3] |
|---|---|---|---|---|---|---|---|---|
| | $^{125}$I-alpha | $^{125}$I-de-alpha | $^{125}$I-IgE/alpha | $^{125}$I-IgE | $^{125}$I-15A5 | $^{125}$I-22E7 | $^{125}$I-IgE | (aa sequence) |
| **Inhibitory (18)[4]** Type 1 (1)[4] (15A5) | + | + | - | + | + | - | - | 125-140 |
| Type 2a (13) (12E7) | + | + | - | + | + | - | - | none |
| Type 2b (3)[5] (6F7) | + | + | - | + | + | - | - | none |
| Type 3 (1) (484) | + | + | - | + | + | - | + | none |
| **Non-Inhibitory (28)** Type 1 (24) (505, 22E7) | + | + | + | - | - | + | - | none |
| Type 2 (3) (39D5) | + | + | + | - | - | - | - | none |
| Type 3 (1) (29C6) | + | + | + | - | +[6] | +[7] | - | none |
| Type 4 (1) (1184) | + | + | + | - | - | + | + | 43-58 |

1) Immunoprecipitation assay (IP Assay) performed as described above with $^{125}$I-labelled purified chimeric α subunit ($^{125}$I-alpha), $^{125}$I-labelled deglycosylated purified chimeric α subunit ($^{125}$I-de-alpha) and the soluble complex of $^{125}$I-IgE crosslinked to chimeric α subunit ($^{125}$I-IgE alpha).

2) Receptor Binding Assay performed as described above with CHO cells expressing recombinant chimeric α subunit [CHO-FcERI(chimeric α subunit)] and RBL cells. The radiolabelled ligands included in the assays were $^{125}$I-IgE, $^{125}$I-15A5 and $^{125}$I-22E7.

3) Peptide EIA performed as described above with 16 synthetic peptides corresponding to the amino

EP 0 499 112 A1

acid sequence (aa sequence) of the extracellular domain of the $\alpha$ subunit.

4) The numbers in parentheses represent the number of antibodies in each "Class" and "Type" of antibody. The prototype antibody is listed in parenthesis below each type of antibody.

5) The distinction between Type 2a and 2b antibodies is that the Type 2b antibodies bind on western blots to the recombinant extracellular domain of the $\alpha$ subunit expressed in E. coli.

6) The $IC_{50}$ is >120 mg/ml.

7) The $IC_{50}$ is 0.18 mg/ml.

The 47 antibodies were divided into non-inhibitory and inhibitory classes depending on their ability to either immunoprecipitate soluble complex of $^{125}$I-IgE cross-linked to chimeric $\alpha$ subunit or to inhibit $^{125}$I-IgE binding to CHO-FcERI(chimeric $\alpha$ subunit) cells, respectively. By SDS/PAGE and autoradiographic analysis, 6 non-inhibitory antibodies (22E7, 29C6, 11B4, 8C8, 24B4 and 5D5) immunoprecipitated two proteins with approximate molecular weights of 190 kD and 250 kD from the soluble extract of $^{125}$I-IgE affinity crosslinked to CHO-FcERI(chimeric $\alpha$ subunit) cells. The inhibitory antibodies 15A5, 12E7, 6F7, 4B4, 23B8, and 39D7 do not immunoprecipitate these radiolabelled complexes. Since the chimeric $\alpha$ subunit is heavily glycosylated, wheat germ lectin bound to cross-linked agarose also precipitates the 190 kD and 250 kD complexes.

Inhibitory antibodies 15A5 and 12E7 block $^{125}$I-IgE binding to CHO-FcERI(chimeric $\alpha$ subunit) cells (Fig. 1). The $IC_{50}$ (half maximal inhibition) to block $^{125}$I-IgE binding are 0.33, 0.89 and 2.43 $\mu g/ml$ for 15A5, IgE and 12E7, respectively. These data suggest that 15A5 and 12E7 have an approximately 2.5 fold higher and lower affinity, respectively, for the $\alpha$ subunit than does IgE. Four non-inhibitory antibodies (22E7, 5D5, 8C8 and 29C6) did not block radiolabelled IgE binding to chimeric $\alpha$ subunit bearing cells (Fig. 1).

The binding of $^{125}$I-15A5, $^{125}$I-IgE and $^{125}$I-22E7 to CHO-FcERI(chimeric $\alpha$ subunit) cells is saturable and specific. Scatchard plot analysis of the equilibrium binding of $^{125}$I-15A5, $^{125}$I-IgE and $^{125}$I-22E7 to CHO-FcERI(chimeric $\alpha$ subunit) cells indicates that the apparent dissociation constants ($K_D$s) for each ligand are 1.57 nM,. 2.99 nM and 0.81 nM, respectively.

Many of the antibodies bind to denatured chimeric $\alpha$ subunit on western blots, but only a subgroup of the non-inhibitory antibodies (i.e.-22E7) bind to both reduced and denatured chimeric $\alpha$ subunit on western blots. The apparent molecular weight of the CHO cell expressed chimeric $\alpha$ subunit is approximately 47 kD as determined by western blot analysis and autoradiography of the $^{125}$I-labelled chimeric $\alpha$ subunit on SDS/PAGE. This apparent molecular weight indicates that the cell surface chimeric $\alpha$ subunit is highly modified posttranslationally since the cDNA for the chimeric $\alpha$ subunit predicts a molecular weight of approximately 24 kD (17). To determine whether the antibodies recognize carbohydrate residues or peptide sequence of the $\alpha$ subunit, each antibody was tested in an immunoprecipitation assay with N-glycanase digested $^{125}$I-labelled chimeric $\alpha$ subunit. The N-glycanase digested receptor migrates with an apparent molecular weight of approximately 25 kD. Both non-inhibitory (11B4 and 39D5) and inhibitory (15A5) antibodies immunoprecipitate the approximately 25 kD deglycosylated $^{125}$I-labelled chimeric $\alpha$ subunit. Most (43 of 47) of the monoclonal antibodies immunoprecipitated deglycosylated $^{125}$I-labelled chimeric $\alpha$ subunit.


k. Identification of Antibody Epitopes.


The binding of each antibody to 16 synthetic peptides spanning and overlapping the entire extracellular sequence minus the signal peptide (amino acids 26 to 204) of the $\alpha$ subunit was determined by EIA. The peptides corresponded to amino acid sequences 26-51, 43-58, 51-65, 59-74, 73-88, 66-80, 81-103, 93-110, 104-117, 111-124, 118-132, 125-140, 134-158, 151-167, 160-197 and 185-204 of the mature $\alpha$ subunit. Of the 43 antibodies tested, only 2 specifically bound to the peptides. Inhibitory antibody 15A5 recognized the peptide spanning amino acids 125-140, while the non-inhibitory antibody 11B4 bound to a peptide representing the sequence 43-58. Neither antibody reacted with any other peptides as for example the peptide corresponding to sequence 81-103. The epitopes of 15A5 and 11B4 probably include the regions around amino acids 130 and 50, respectively, since neither antibody binds to the partially overlapping peptides (118-132 and 132-158 for 15A5; 26-51 and 51-65 for 11B4).

To identify the epitopes of the antibodies which did not bind to the synthetic peptides, competition studies were performed which measured the ability of each antibody to block $^{125}$I-15A5 and $^{125}$I-22E7 (a homologue of antibody 11B4) binding to the CHO-FcERI(chimeric $\alpha$ subunit) cells. The binding of $^{125}$I-15A5 and $^{125}$I-22E7 to the cellular receptor was saturable and specific. The $IC_{50}$s for inhibition of $^{125}$I-15A5 binding by unlabelled 15A5, IgE and 12E7 were 1.09, 2.41 and 13.74 mg/ml, respectively (Fig. 2). All of the neutralizing antibodies could block $^{125}$I-15A5 binding to CHO-FcERI(chimeric $\alpha$ subunit) cells, suggesting that the neutralizing antibodies recognized a common epitope or closely adjacent epitopes. One non-inhibitory antibody, 29C6, could partially inhibit $^{125}$I-15A5 binding at 120 $\mu g/ml$, whereas the other non-inhibitory antibodies did not block $^{125}$I-15A5 binding at these concentrations (Fig.2).

The $IC_{50}$s for inhibition of $^{125}$I-22E7 binding by unlabelled 22E7, 29C6, 8C8 and 5D5 were 0.26, 0.18, 0.29, and 0.79 $\mu$g/ml, respectively. Hybridoma supernatants containing 22 of the 25 non-neutralizing antibodies blocked $^{125}$I-22E7 binding to CHO-FcERI(chimeric $\alpha$ subunit) cells. The epitopes recognized by the remaining three non-neutralizing antibodies (i.e. 39D5) did not block radiolabelled 22E7 binding to these cells indicating that their epitopes were not overlapping with the 22E7 epitope (amino acid sequence 43-58). The inhibitory antibody 15A5 blocked approximately 25% of radiolabelled 22E7 binding to the CHO-FcERI-(chimeric $\alpha$ subunit) cells. This level of inhibition was constant and could not be increased by adding more 15A5 to the binding reaction. Unlabelled IgE and the other inhibitory antibodies (i.e. 12E7) could not inhibit radiolabelled 22E7 binding to these cells.

1. Antibody Binding to the Native FcE Receptor (FcERI) on Human Basophils.

Two color immunofluorescence assays were performed to evaluate the ability of the monoclonal antibodies of the present invention to recognize the native $\alpha$ subunit as part of the FcERI complex on human basophils. Since normal human basophils usually have IgE bound to the majority of their FcE receptors, FITC labelled anti-IgE will stain the basophils indicating the presence of cells with IgE occupied FcERI receptors. The basophils are very faintly stained with inhibitory antibody 15A5. This antibody brightly stains CHO-FcERI(chimeric $\alpha$ subunit) cells and shows only faint staining to these same cells after pretreatment with IgE . These data indicate that the 15A5 epitope is blocked on the majority of FcERI receptors on the basophils. However, the non-inhibitory antibody 5D5 brightly stains the same cells to which IgE is bound, indicating that IgE and 5D5 can simultaneously bind to the native FcERI receptor. Both antibodies only stained anti-IgE stained cells demonstrating that the antibodies are highly specific for basophils.

The FcERI receptor promotes degranulation of basophils when IgE and antigen crosslink two or more receptors. Bivalent antibody molecules also promote degranulation of basophils by crosslinking the native FcERI receptor. The ability of each antibody to induce degranulation can be measured by the amount of histamine released from the basophils when in the presence of different concentrations of each antibody (Fig. 3). Anti-IgE antibodies induce histamine release from the basophils in a dose dependent manner (Fig. 3, inset panel). The non-inhibitory antibodies, 5D5 and 22E7, can induce maximal histamine release at the lowest antibody concentrations (Fig. 3). However, inhibitory antibodies, 15A5 and 12E7, do not induce histamine release at the lowest antibody concentration (Fig. 3), because most FcERI receptors are occupied by IgE. Higher concentrations of these two neutralizing antibodies do induce histamine release (Fig. 3) most likely due to crosslinking of the receptors not occupied by IgE.

m. Antibody Binding to Native FcERI Receptor on RBL Cells.

Six inhibitory (15A5, 4B4, 39D7, 12E7, 6F7 and 23B8) and six non-inhibitory (22E7, 5D5, 8C8, 24B4, 29C6 and 11B4) antibodies were tested for their ability to inhibit $^{125}$I-IgE binding to the FcERI receptor on RBL cells or to immunoprecipitate the soluble complex of $^{125}$I-IgE crosslinked to the rat $\alpha$ subunit. Only one inhibitory antibody, 4B4, blocks $^{125}$I-rat IgE binding to RBL cells. The $IC_{50}$s for 4B4 blocking $^{125}$I-IgE binding to CHO-ERI(chimeric $\alpha$ subunit) cells and RBL cells are 0.6 and 330 mg/ml, respectively. Unlabelled rat IgE was approximately 1100 times more potent than 4B4 at blocking $^{125}$I-rat IgE binding to RBL cells. No other antibody could block $^{125}$I-rat IgE binding to the RBL cells although these antibodies were very potent at blocking $^{125}$I-human IgE binding to CH0-FcERI(chimeric $\alpha$ subunit) cells. None of the non-inhibitory antibodies could immunoprecipitate the soluble complex of $^{125}$I-IgE crosslinked to rat $\alpha$ subunit.

n. Preparation and Use of Fab Fragments of Monoclonal Antibodies

Fab fragments of purified monoclonal antibodies of the present invention were prepared by papain digestion according to well known and standard techniques as described in "Antibodies: A Laboratory Manual", E. Harlow and D. Lane, eds., Cold Spring Harbor Press, 1989; pp. 625-631 and in "Monoclonal Antibodies: Principles and Practice", J. Goding, Academic Press, Inc., New York, N.Y., 119-124 (1983).

Fab fragments of purified monoclonal antibodies can be used to block IgE binding to human basophils and mast cells by the method described for the intact antibody molecules. Demonstration of inhibition of IgE binding to basophils and mast cells by the Fab fragments without stimulation of degranulation and histamine release has been performed by standard techniques as described in Progress in Allergy, Vol. 34, pp. 188-253 "Activation of Mast Cells for Mediator Release through IgE Receptors", T. I. Ishizaka and K. Ishizaka.

REFERENCES

1. Metzger, H., Alcaraz, G., Hahman, R., J.P. Kinet, Publuda V., and Quarto, R. (1986) Annu. Rev. Immunol. 4, 419-470

2. Conrad, D.H., Berczi, I., and Froese, A. (1976) Immunochemistry 13, 329-332

3. Kulczycki, A.,Jr., McNearney, T.A., and Parker, C.W. (1976) J. Immunol. 117, 661-665

4. Holowka, D., Hartmann, H., Kannelopoulos, J., and Metzger, H. (1980) J. Recept. Res. 1, 41-68

5. Holowka, D., and Metzger, H. (1982) Mol. Immunol. 19, 219-2276. Perez-Montfort, R., Kinet, J.-P., and Metzger, H. (1983) Biochemistry 22, 5722-5728

7. Perex-Montfort, R., Fewtrell, C., and Metzger, H. (1983) Biochemistry 22, 5733-5737

8. Alcaraz, G., Kinet, J.-P., Kumar, N., Wank, S.A., and Metzger, H. (1984) J. Biol. Chem. 259, 14922-14927

9. Kinet, J.-P., Metzger, H., Hakimi, J., and Kochan, J. (1987) Biochemistry 26, 4605-4610

10. Shimizu, A., Tepler, I., Benfey, P.N., Berenstein, E., Siraganian, R.P., and Leder, P. (1988) Proc. Natl. Acad. Sci. USA 85, 1907-1911

11. Kinet, J.-P., Blank, U., Ra, C., White, K., Metzger, H., and Kochan, J. (1988) Proc. Natl. Acad. Sci. USA 85, 6483-6487

12. Blank, U., Ra, C., Miller, L, White, K., Metzger, H., and Kinet, J.-P. (1989) Nature 337, 187-189

13. Ra, C., Jouvin, M.-H. E., and Kinet, J.-P. (1989) J. Biol. Chem. 264, 15323-15327

14. Kochan, J., Pettine, L.F., Hakimi, J., Kishi, K. and Kinet, J.-P. (1988) Nucleic Acids Res. 16, 3584

15. Kuster, H., Thompson, H., and Kinet, J.-P. (1990) J. Biol. Chem 265: 6448

16. Miller, L., Blank, U. Metzger, H., and Kinet, J.-P. (1989) Science 244, 334-337

17. Hakimi, J., Seals, C., Pettine, L.F. and Kochan, J. (1990) Science (in press)

18. Ishizaka, K. (1985) Immunoglobulin E (IgE). Methods In Enzymology 116, 76

19. Bazin, H., Querinjean, P., Beckers, A., Heremans, J. F., and Dessy, F. (1974) Immunology 26, 713

20. Bayer, E.A., Skutelsky, E., and Wilchek, M. (1979) Methods in Enzymology 62, 308

21. McConahey, P., and Dixon, F.J. (1986) Int. Arch. Allergy 29, 185

22. Kulczycki, A., Jr., Isersky, C., and Metzger, H. (1974) J. Exp. Med. 139, 600

23. Kilian, P.L., Kaffka, K.L, Stern., A.S., Woehle, D., Benjamin, W.R., Dechiara, T.M., Gubler, U., Farrar,J., Mizel, S.B., and Lomedico, P.T. (1986) J. Immunol. 136, 1-6

24. Laemmli, J.K. (1970) Nature 227, 680-685

25. Galfre, G., and Milstein, C. (1981) Methods in Enzymology 73, 3-466.

26. Fazekas De St. Groth, S, and Scheidegger, D. (1980) J. Immunol. Methods 35, 1-21

27. Nordan, R.P., Pumphrey, J.G., and Rudikoff, S. (1987) J. Immunol. 139, 813-817

28. Burnette, W.H. (1981) Anal. Biochem. 112, 195-200

29. Barany, G., and Merrifield, R.B. (1980) The Peptides: Analysis Synthesis, Biology (Gross, E., and Meienhofer, J., eds) Vol. 2, pp. 1-255, Academic Press, New York.

30. Scatchard, G. (1949) Ann. N.Y. Acad. Sci. 51, 660-672

31. Leonard E.J., Roberts, R.L., and Skeel, A. (1984) J. Leukocyte Biol. 35, 169-

32. Krenik, K.D., Kephart, G.M., Offord, K.P., Dunnette, S.L., and Gleich, G.J. (1989) J. Immunol. Methods 117, 91-97

33. Helm, B., Kebo, D., Verchelli, D., Glorsky, M., Gould, H., Ishizaka, K., Gehe, R., and Ishizaka, T. (1989) Proc. Natl. Acad. Sci. USA 86, 9465-9469

34. Ravetch, J.V., Luster, A.D., Weinshank, R., Kochan, J., Pavlovec, A., Portnoy, D.A., Hulmes, J., Pan, Y.-C.E., and Unkeles, J.C. (1986) Science 234, 718-725

35. Basciano, L.K., Berenstein, E.H., Kmak, L., and Siraganian, R.P. (1986) J. Biol. Chem. 261, 11823-11831

36. Baniyash, M., Alkalay, I., and Eshhar, Z. (1987) J. Immunol. 138, 2999-3004

37. Stracke, M.L., Basciano, L.K., Fischler, C., Berenstein, E.H., and Siraganian, R.P. (1987) Mol. Immunol. 24, 347-356

38. Miroli, A.A., and Spitz, M. (1987) J. Immunol. Methods 97, 185-190

39. Guyre, P.M., Graziano, R.R., Vance, B.A., Morganelli, P.M., and Fanger, M.W. (1989) J. Immunol. 143, 1650-1655

40. Nisonoff, A. (1982) Introduction to Molecular Immunology, Sinauer Associates Inc., Sunderland, Mass.

41. Harlow, E. and Lane, D., eds (1989), Antibodies, A Laboratory Manual, Cold Spring Harbor Press, 625-631.

42. Goding, J. (1983) Monoclonal Antibodies: Principles and Practice, New York, Academic Press, Inc., 119-124.

43. Ishizaka, T. and Ishizaka K., Progress in Allergy, 34, 188-235.

44. Lawlor E., W. Collins, and B. O'Connor. (1986). A case of IgE Myeloma. Clin. Chem. 32:697.

45. Lui, F.T., M. Sinnecker, J. Hamaoka, and D. Katz. (1979). Biochemistry 18:690.

46. Marinaga, Y., T. Franceschiri, S. Inouye, and M. Inouye. (1984). Improvement of oligonucleotide-directed site-specific mutagenesis using double-strand plasmid. DNA Biotechnol. 2:636.

47. Cullen, B. (1987). Use of eukaroytic expression technology in the functional analysis of cloned genes. Methods in Enzymology 152:684.

48. Nikaido, T., A. Shimizu, N. Ishida, H. Sabe, K. Teshigawara, M. Maeda, T. Uchiyama, J. Yodoi, and T. Honjo. (1984). Molecular cloning of cDNA encoding human interleukin-2 receptor. Nature 311:631.

49. Isersky, C., A. Kulczycki, Jr., and H. Metzger. (1974). Isolation of IgE from reaginic rat serum. J. Immunol. 112:1909.

50. Kulczycki, A., Jr., and H. Metzger. (1974). The interaction of IgE with rat basophilic leukemia cells. II. Quantitative aspects of the binding reaction. J. Exp. Med. 140:167.

51. Ishizaka, T., B. Helm, J. Hakimi, J. Niebyl, K. Ishizaka, and H. Gould. (1986). Biological properties of a recombinant human immunoglobulin epsilon-chain fragment. Proc. Natl. Acad. Sci. USA 83:8323.

52. Ishizaka, T., A. M. Dvorak, D. H. Conrad, J. R. Niebyl, J. P. Marquette, and K. Ishizaka. (1985). Morphologic and immunologic characterization of human basophils developed in cultures of cord blood mononuclear cells. J. Immunol. 134:532.

53. Conrad, D. H., J. R. Wingard, and T. Ishizaka. (1983). The interaction of human and rodent IgE with the human basophil IgE receptor. J. Immunol. 130:327

## Claims

1. A monoclonal antibody or a fragment thereof capable of specifically binding to at least one epitope of the α subunit of the human high affinity Fc IgE receptor.

2. A fragment according to claim 1 which is a mono- or bivalent Fab fragment.

3. A monoclonal antibody or a fragment thereof according to claim 1 or 2 whereby the amino acid sequence of the α subunit of the human high affinity Fc IgE receptor is as follows:

```
Val Pro Gln Lys Pro Lys Val Ser Leu Asn Pro Pro Trp Asn

Arg Ile Phe Lys Gly Glu Asn Val Thr Leu Thr Cys Asn Gly Asn

Asn Phe Phe Glu Val Ser Ser Thr Lys Trp Phe His Asn Gly Ser

Leu Ser Glu Glu Thr Asn Ser Ser Leu Asn Ile Val Asn Ala Lys

Phe Glu Asp Ser Gly Glu Tyr Lys Cys Gln His Gln Gln Val Asn

Glu Ser Glu Pro Val Tyr Leu Glu Val Phe Ser Asp Trp Leu Leu

Leu Gln Ala Ser Ala Glu Val Val Met Glu Gly Gln Pro Leu Phe

Leu Arg Cys His Gly Trp Arg Asn Trp Asp Val Tyr Lys Val Ile

Tyr Tyr Lys Asp Gly Glu Ala Leu Lys Tyr Trp Tyr Glu Asn His

Asn Ile Ser Ile Thr Asn Ala Thr Val Glu Asp Ser Gly Thr Tyr

Tyr Cys Thr Gly Lys Val Trp Gln Leu Asp Tyr Glu Ser Glu Pro

Leu Asn Ile Thr Val Ile Lys Ala Pro Arg Glu Lys
```

4. A monoclonal antibody or a fragment thereof according to any one of claims 1-3 which blocks IgE binding to the high affinity Fc IgE receptor of a human basophil or a human mast cell.

5. A monoclonal antibody or a fragment thereof according to any one of claims 1-3 which does not stimulate histamine release when bound to the α subunit of a high affinity Fc IgE receptor of a human basophil or mast cell.

6. A monoclonal antibody or a fragment thereof according to any one of claims 1-5 which is of mammalian origin.

7. A hybridoma cell secreting a monoclonal antibody according to any one of claims 1-6.

8. A monoclonal antibody or a fragment thereof according to any one of claims 1-6 as a therapeutically active substance.

9. A monoclonal antibody or a fragment thereof according to any one of claims 1-6 as a diagnostic tool.

10. A monoclonal antibody or a fragment thereof according to any one of claims 1-6 for the purification of the α subunit of the human high affinity Fc IgE receptor.

11. A process for the preparation of a hybridoma cell which produces monoclonal antibodies as claimed in any one of claims 1-6 which process comprises:
   (a) injecting a polypeptide comprising a first amino acid sequence corresponding to the α subunit of the human high affinity Fc IgE receptor or any functional subsequence thereof and a second amino acid sequence corresponding to a transmembrane and cytoplasmic region of a receptor which allows cell surface expression of said α subunit mixed with an adjuvant into a mammal, and repeating the injection at least once so as to produce an immune response;
   (b) isolating antibody-producing cells from said mammal;
   (c) fusing said cells with myeloma cells; and
   (d) selecting from the resulting hybridoma cells those cells which produce the desired monoclonal antibody.

12. A process for the preparation of a monoclonal antibody or a fragment thereof as claimed in anyone of claims 1-6 which process is characterized by the steps (a) to (d) according to claim 11 and a step (e) comprising isolating the desired monoclonal antibody from the supernatant of the corresponding hybridoma cell and, if desired, preparing a fragment thereof by methods known in the art.

13. A process for purifying the α subunit of the human high affinity Fc IgE receptor which process comprises:
   (a) contacting a sample with the monoclonal antibody or fragment optionally coupled to a solid support under conditions such that a complex is formed between said monoclonal antibody or fragment, and said α subunit present in the sample;
   (b) separating the complex formed from the sample; and
   (c) isolating from the complex said α subunit in purified form.

14. A process for detecting the presence of the α subunit of the human high affinity Fc IgE receptor in a sample which process comprises:
   (a) contacting a sample optionally a biological fluid with the monoclonal antibody or fragment thereof as defined in any one of claims 1-6 labelled with a detectable marker optionally an enzyme or a radioactive or fluorescent entity to form a complex between the monoclonal antibody or fragment and said α subunit present in the sample; and
   (b) detecting labelled complex and thereby detecting the presence of said α subunit in the sample.

15. A process for detecting the presence of the α subunit of the human high affinity Fc IgE receptor in a sample which process comprises:
   (a) contacting a sample with a monoclonal antibody or fragment thereof as defined in any one of claims 1-6 to form a complex between the monoclonal antibody or fragment, and said α subunit present in the sample;
   (b) contacting the resulting sample with a detectably labelled antibody capable of binding to said monoclonal antibody or fragment to form a complex between the detectably labelled monoclonal antibody and the complex of step (a); and

(c) detecting labelled complex of step (b) and thereby detecting the presence of said α subunit in the sample.

**16.** A process for determining the amount of IgE present in a sample capable of binding to the α subunit of the human high-affinity Fc IgE receptor which process comprises:

(a) contacting a monoclonal antibody or fragment thereof as defined in any one of claims 1-6 coupled to a solid support with a known amount of isolated α subunit of the human high-affinity Fc IgE receptor to form a complex between the monoclonal antibody or fragment and said α subunit;

(b) contacting the complex of step (a) with a sample to bind IgE in the sample to said α subunit of said complex to form a new complex;

(c) contacting the newly formed complex of step (b) with a known amount of a detectably labelled antibody capable of binding to IgE; and

(e) determining the amount of IgE present in the sample by measuring the amount of detectably labelled antibody of step (c).

**17.** A pharmaceutical composition containing a monoclonal antibody or a fragment thereof according to any one of claims 1-6 and if desired a non-toxic, inert therapeutically acceptable carrier material.

**18.** The use of a monoclonal antibody or a fragment thereof according to any one of claims 1-6 as a therapeutic agent.

**19.** The use of a monoclonal antibody or a fragment thereof according to any one of claims 1-6 as a therapeutic agent for the treatment of allergic diseases.

**20.** The use of a monoclonal antibody or a fragment thereof according to any one of claims 1-6 as a diagnoatic agent.

**21.** The use of a monoclonal antibody or a fragment thereof according to any one of claims 1-6 for the purification of the α-subunit of the human high-affinity Fc IgE receptor.

**Claims for the following Contracting States : GR, ES**

**1.** A process for the preparation of a monoclonal antibody or a fragment thereof capable of specifically binding to at least one epitope of the α subunit of the human high affinity Fc IgE receptor which process comprises:

(a) injecting a polypeptide comprising at least one epitope of the α subunit of the human high affinity Fc IgE receptor mixed with an adjuvant into a mammal, and repeating the injection at least once so as to produce an immune response;

(b) isolating antibody-producing cells from said mammal;

(c) fusing said cells with myeloma cells;

(d) selecting from the resulting hybridoma cells those cells which produce the desired monoclonal antibody; and

(e) isolating the desired monoclonal antibody from the supernatant of the corresponding hybridoma cell and if desired, preparing a fragment thereof by methods known in the art.

**2.** A process as claimed in claim 1 whereby the polypeptide used in step a) of said claim comprises a first amino acid sequence corresponding to the α subunit of the human high affinity Fc IgE receptor or any functional subsequence thereof and a second amino acid sequence corresponding to a transmembrane and cytoplasmic region of a receptor which allows cell surface expression of said α subunit.

**3.** A process as claimed in claim 1 or 2 whereby the fragment is a mono- or bivalent Fab fragment.

**4.** A process as claimed in any one of claims 1-3 whereby the amino acid sequence of the α subunit of the human high affinity Fc IgE receptor is as follows:

```
        Val Pro Gln Lys Pro Lys Val Ser Leu Asn Pro Pro Trp Asn
      Arg Ile Phe Lys Gly Glu Asn Val Thr Leu Thr Cys Asn Gly Asn
      Asn Phe Phe Glu Val Ser Ser Thr Lys Trp Phe His Asn Gly Ser
      Leu Ser Glu Glu Thr Asn Ser Ser Leu Asn Ile Val Asn Ala Lys
      Phe Glu Asp Ser Gly Glu Tyr Lys Cys Gln His Gln Gln Val Asn
      Glu Ser Glu Pro Val Tyr Leu Glu Val Phe Ser Asp Trp Leu Leu


      Leu Gln Ala Ser Ala Glu Val Val Met Glu Gly Gln Pro Leu Phe
      Leu Arg Cys His Gly Trp Arg Asn Trp Asp Val Tyr Lys Val Ile
      Tyr Tyr Lys Asp Gly Glu Ala Leu Lys Tyr Trp Tyr Glu Asn His
      Asn Ile Ser Ile Thr Asn Ala Thr Val Glu Asp Ser Gly Thr Tyr
      Tyr Cys Thr Gly Lys Val Trp Gln Leu Asp Tyr Glu Ser Glu Pro
      Leu Asn Ile Thr Val Ile Lys Ala Pro Arg Glu Lys
```

5. A process as claimed in any one of claims 1-4 whereby said monoclonal antibody or a fragment thereof blocks IgE binding to the high affinity Fc IgE receptor of a human basophil or a human mast cell.

6. A process as claimed in any one of claims 1-4 whereby said monoclonal antibody or a fragment thereof does not stimulate histamine release when bound to the α subunit of a high affinity Fc IgE receptor of a human basophil or mast cell.

7. A process as claimed in any one of claims 1-6 whereby said monoclonal antibody or a fragment thereof is of mammalian origin.

8. A process for the preparation of a hybridoma cell secreting a mono-clonal antibody prepared by a process as claimed in any one of claims 1-7 whereby said process consists of steps a)-d) of claim 1.

9. A process for the preparation of a pharmaceutical composition which process is characterized in that a compound obtained by a process as claimed in any one of claims 1-7 and if desired, additional pharmaceutically active substances are mixed with a non-toxic, inert, therapeutically compatible carrier material and the mixture is brought into a galenical application form.

10. A pharmaceutical composition which contains one or more compounds obtained according to a process as claimed in any one of claims 1-7, if desired, in combination with additional pharmaceutically active substances and/or non-toxic, inert, therapeutically compatible carrier materials.

11. The use of a compound prepared according to a process as claimed in any one of claims 1-7 for the preparation of a pharmaceutical composition according to claim 10.

12. A compound whenever prepared according to a process as claimed in any one of claims 1-8.

13. A process for purifying the α subunit of the human high affinity Fc IgE receptor which process comprises:
(a) contacting a sample with a monoclonal antibody or fragment thereof obtained according to a process as claimed in any one of claims 1-7, optionally coupled to a solid support, under conditions such that a complex is formed between said monoclonal antibody or fragment, and said α subunit present in the sample;
(b) separating the complex formed from the sample; and

23

(c) treating the complex to purify said α subunit therefrom.

14. A process for detecting the presence of the α subunit of the human high affinity Fc IgE receptor in a sample which process comprises:

(a) contacting a sample, optionally a biological fluid, with a monoclonal antibody or fragment thereof, obtained according to a process as claimed in any one of claims 1-7, labelled with a detectable marker optionally an enzyme or a radioactive or fluorescent entity to form a complex between the monoclonal antibody or fragment and said α subunit present in the sample; and

(b) detecting labelled complex and thereby detecting the presence of said α subunit in the sample.

15. A process for detecting the presence of the α subunit of the human high affinity Fc IgE receptor in a sample which process comprises:

(a) contacting a sample with a monoclonal antibody or fragment thereof obtained according a process as claimed in any one of claims 1-7 to form a complex between the monoclonal antibody or fragment, and said α subunit present in the sample;

(b) contacting the resulting sample with a detectably labelled antibody capable of binding to said monoclonal antibody or fragment to form a complex between the detectably labelled monoclonal antibody and the complex of step (a); and

(c) detecting labelled complex of step (b) and thereby detecting the presence of said α subunit in the sample.

16. A process for determining the amount of IgE present in a sample capable of binding to the α subunit of the human high-affinity Fc IgE receptor which process comprises:

(a) contacting a monoclonal antibody or fragment thereof obtained according to a process as claimed in any one of claims 1-7 coupled to a solid support with a known amount of isolated α subunit of the human high-affinity Fc IgE receptor to form a complex between the monoclonal antibody or fragment and said α subunit;

(b) contacting the complex of step (a) with a sample to bind IgE in the sample to said α subunit of said complex to form a new complex;

(c) contacting the newly formed complex of step (b) with a known amount of a detectably labelled antibody capable of binding to IgE; and

(d) determining the amount of IgE present in the sample by measuring the amount of detectably labelled antibody of step (c).

17. The inventions as hereinbefore described.

18. A hybridoma cell whenever prepared by a process as claimed in claim 8.

Fig. 1

Fig. 2

Fig. 3

Fig. 4.

European Patent Office

# PARTIAL EUROPEAN SEARCH REPORT

which under Rule 45 of the European Patent Convention shall be considered, for the purposes of subsequent proceedings, as the European search report

Application Number

EP 92 10 1732

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | WO-A-8 905 352 (PRESIDENT AND FELLOWS OF HARVARD COLLEGE) * Page 7, line 25 - page 8, line 26 * --- | 1-21 | C 12 P 21/08 C 12 N 5/20 C 12 N 15/06 G 01 N 33/577 G 01 N 33/68 A 61 K 39/395 |
| D,X | JOURNAL OF IMMUNOLOGICAL METHODS, vol. 97, no. 2, 12th March 1987, pages 185-190, Amsterdam, NL; A.A. MIROLI et al.: "Studies on the human basophil IgE receptor, generation and characterization of monoclonal antibodies against the receptor" * The whole document * --- | 1-7,9-16,20,21 | |
| X | THE JOURNAL OF BIOLOGICAL CHEMISTRY, vol. 265, no. 36, 25th December 1990, pages 22079-22081, Baltimore, MD, US; J. HAKIMI et al.: "The alpha subunit of the human IgE receptor (FcERI) is sufficient for high affinity IgE binding" * Page 22080, left-hand column, lines 21-45; figure 1 * --- -/- | 1-21 | TECHNICAL FIELDS SEARCHED (Int. Cl.5) C 12 P C 12 N G 01 N A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

Remark: Although claims 18 and 19 are directed to a method of treatment of the human/animal body, the search has been carried out and based on the alleged effects of the compound/composition.

| Place of search | Date of completion of the search | Examiner |
|---|---|---|
| THE HAGUE | 29-04-1992 | NOOIJ F.J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

European Patent
Office

**PARTIAL EUROPEAN SEARCH REPORT**

| | DOCUMENTS CONSIDERED TO BE RELEVANT | | CLASSIFICATION OF THE APPLICATION (Int. Cl.5) |
|---|---|---|---|
| Category | Citation of document with indication, where appropriate, of relevant passages | Relevant to claim | |
| A | THE JOURNAL OF BIOLOGICAL CHEMISTRY, vol. 266, no. 3, 25th January 1991, pages 1903-1909, Baltimore, MD, US; S. KITANI et al.: "A cell surface glycoprotein of rat basophilic leukemia cells close to the high affinity IgE receptor (FcERI)" * Page 1904, left-hand column, lines 35-43 * | 1-7,9-16,20, 21 | |
| P,X | THE JOURNAL OF BIOLOGICAL CHEMISTRY, vol. 266, no. 17, 15th June 1991, pages 11245-11251, Baltimore, MD, US; F. RISKE et al.: "High affinity human IgE receptor (FcERI). Analysis of functional domains of the alpha-subunit with monoclonal antibodies" * Abstract * | 1-7,9-16,20, 21 | TECHNICAL FIELDS SEARCHED (Int. Cl.5) |
| P,X | JOURNAL OF CELLULAR BIOCHEMISTRY, suppl. 15, part E, 1991, page 127, abstract no. 208, New York, US; M. GRIFFIN et al.: "High affinity human IgE receptor (FcERI): Analysis of the functional domains of the alpha-subunit with monoclonal antibodies" * Abstract * | 1-7,9-16,20, 21 | |

EPO FORM 1503 03.82 (P0410)